(19)

**Europäisches Patentamt**
**European Patent Office**
**Office européen des brevets**

(11)   **EP 2 615 117 B2**

(12)   # NEW EUROPEAN PATENT SPECIFICATION
After opposition procedure

(45) Date of publication and mention
of the opposition decision:
**27.12.2023   Bulletin 2023/52**

(45) Mention of the grant of the patent:
**17.03.2021   Bulletin 2021/11**

(21) Application number: **11823500.1**

(22) Date of filing: **02.09.2011**

(51) International Patent Classification (IPC):
*C08F 2/20* (2006.01)     *A61F 13/53* (2006.01)
*A61L 15/60* (2006.01)     *C08F 20/06* (2006.01)
*C08F 2/32* (2006.01)      *B01J 20/28* (2006.01)
*A61L 15/22* (2006.01)

(52) Cooperative Patent Classification (CPC):
**B01J 20/28016; A61L 15/22; A61L 15/60;**
**C08F 2/32; C08F 20/06;** A61F 2013/530481

(86) International application number:
**PCT/JP2011/070068**

(87) International publication number:
**WO 2012/033025 (15.03.2012 Gazette 2012/11)**

(54)   **WATER ABSORBENT RESIN AND METHOD FOR PRODUCING SAME**

WASSERABSORBIERENDES HARZ UND VERFAHREN ZU SEINER HERSTELLUNG

RÉSINE ABSORBANT L'EAU ET SON PROCÉDÉ DE PRODUCTION

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority:  **06.09.2010   JP 2010198836**

(43) Date of publication of application:
**17.07.2013   Bulletin 2013/29**

(73) Proprietor: **SUMITOMO SEIKA CHEMICALS CO.,**
**LTD.**
**Kako-gun, Hyogo 675-0145 (JP)**

(72) Inventors:
• **HANDA, Masayoshi**
**Himeji-shi**
**Hyogo 672-8076 (JP)**
• **ONODA, Yuichi**
**Himeji-shi**
**Hyogo 672-8076 (JP)**
• **UEDA, Koji**
**Himeji-shi**
**Hyogo 672-8076 (JP)**

(74) Representative: **Vossius & Partner**
**Patentanwälte Rechtsanwälte mbB**
**Siebertstrasse 3**
**81675 München (DE)**

(56) References cited:
EP-A1- 0 441 507          EP-A1- 0 789 047
EP-A1- 0 789 048          EP-A1- 0 812 873
EP-A1- 1 142 696          EP-A1- 2 011 803
EP-A1- 2 011 803          EP-A1- 2 184 300
EP-A1- 2 674 441          EP-A2- 0 349 241
WO-A1-2005/012406         WO-A1-2006/014031
WO-A1-2006/014031         WO-A1-2007/123188
WO-A1-2007/123188         WO-A2-2005/011860
JP-A- 3 195 713           JP-A- 3 227 301
JP-A- 3 227 301           JP-A- 2001 002 935
US-A- 5 976 696           US-A1- 2007 015 860
US-A1- 2007 066 754

• **Experimental report reproducing Example 13 of**
**D1**
• **Fredric L. Buchholz, Et Al.: "Modern**
**Superabsorbent Polymer Technology" In:**
**"Modern Superabsorbent Polymer Technology",**
**1 January 1998 (1998-01-01) pages 69-117,**

Remarks:
The file contains technical information submitted after
the application was filed and not included in this
specification

EP 2 615 117 B2

**Description**

Technical Field

The present invention relates to a water-absorbent resin. Specifically, the present invention relates to a water-absorbent resin having properties suitable for a water-absorbent agent used in a hygienic material, such as a suitable particle size, strong particle strength, excellent absorption properties, excellent adhesion to fibers, and excellent flowability as powders. The present invention also relates to an absorbent material using the water-absorbent resin, and an absorbent article such as a disposable diaper.

Background Art

In recent years, a water-absorbent resin has been widely used in various fields, including hygienic articles, such as disposable diapers and sanitary napkins, agricultural and horticultural materials, such as water-retaining materials and soil conditioners, and industrial materials, such as waterblocking agents and agents for preventing dew condensation. Of these, a water-absorbent resin is particularly frequently used in hygienic articles, such as disposable diapers and sanitary napkins. As the water-absorbent resin, for example, hydrolysates of starch-acrylonitrile graft copolymers, neutralized products of starch-acrylate graft copolymers, saponified products of vinyl acetate-acrylic ester copolymers, and crosslinked polymers of partially neutralized acrylic acid compound are known.

In general, properties desired for the water-absorbent resin include a large amount of water absorption, an excellent water-absorption rate, and a high gel strength after water absorption.

In particular, in addition to a large amount of water absorption, an excellent water-absorption rate, and a high gel strength after water absorption, properties desired for the water-absorbent resin used in an absorbent material applied to a hygienic material include an excellent water absorption capacity under load, an appropriate particle size, a narrow particle size distribution, a small re-wet amount of the absorbed substance to the external of the absorbent material, and an excellent dispersibility of the absorbed substance to the internal of the absorbent material.

Absorbent articles such as disposable diapers have a structure in which an absorbent material for absorbing a liquid, e.g., body liquid, is sandwiched between a flexible liquid-permeable surface sheet (top sheet) positioned on a side contacting the body and a liquid-impermeable rear sheet (back sheet) positioned on a side opposite to that contacting the body.

In recent years, there is increasing demand for thinner and lighter absorbent articles from the viewpoint of design, portability convenience, and transport efficiency. A generally employed method for thinning absorbent articles is, for example, a method in which the amount of hydrophilic fiber, such as refined wood pulp, which serves to secure a water-absorbent resin in an absorbent material, is reduced, and the amount of water-absorbent resin is increased.

An absorbent material that contains a reduced amount of a bulky hydrophilic fiber having a low absorption capacity and an increased amount of a compact water-absorbent resin having a high absorption capacity is intended to reduce the thickness of the absorbent material by reducing the amount of the bulky material while keeping the absorption capacity that corresponds to the design of the absorbent article, and this is a reasonable improvement method. However, when liquid distribution or diffusion that occurs in the actual use of an absorbent material in an absorbent article, e.g., a disposable diaper, is considered, there is a disadvantage in that when a large amount of the water-absorbent resin is formed into a soft gel by liquid absorption, a phenomenon called "gel blocking" occurs, which markedly lowers liquid diffusibility and slows the liquid permeation time of the absorbent material. This gel blocking phenomenon is explained below. When an absorbent material containing particularly highly densified water-absorbent resins absorbs liquid, a water-absorbent resin near the surface layer absorbs the liquid to further densify a soft gel around the surface layer, and so liquid permeation into the absorbent material is inhibited, preventing the internal water-absorbent resins from efficiently absorbing the liquid.

As a means for inhibiting the gel blocking phenomenon, which occurs when the amount of hydrophilic fiber is reduced and a large amount of water-absorbent resin is used, methods proposed so far include a method using an absorption polymer having a saline flow conductivity and a performance under pressure (Patent Literature 1), and a method using a water-absorbent resin in which a water-absorbent resin precursor is heat-treated using a surface cross-linking agent (Patent Literature 2). However, in these methods, an absorbent material in which a water-absorbent resin is used in a large amount cannot attain sufficient absorption properties.

Considering convenience in the manufacturing process of absorbent articles such as disposable diapers and sanitary napkins and the absorption stability of absorbent articles, features associated with the configuration of a water-absorbent resin, i.e., the particle size, powder flowability, and impact resistance strength of a water-absorbent resin become important factors in addition to the aforementioned absorption properties. In general, absorbent materials for use in absorbent articles are produced in equipment generally called a "drum former". In the equipment, a water-absorbent resin is supplied to a refined fibrous pulp using a powder transfer device such as a screw feeder to mix the resin and pulp in air, and the result is suctioned and laminated on a metal screen mesh to form an absorbent material. Thereafter, to increase a shape retention property, the absorbent material is compressed with a roll press, thereby incorporating it into an absorbent article.

In an absorbent material production process, when a water-absorbent resin has an inappropriate particle size, specifically, a small particle size, dusting occurs around the manufacturing equipment, or the resin can pass through mesh, which lowers the production efficiency. Further, when the water-absorbent resin has poor powder flowability, the supply amount varies in a powder transfer device, and a bridge formed at the inlet is likely to cause variations in the properties of absorbent articles. Furthermore, the water-absorbent resin having low impact resistance is easily broken when it collides with mesh or is compressed with a press. A water-absorbent resin of larger particle size, especially in case of increasing its size by agglomeration, tends to be weak against the impact.

Mainly because the amount of hydrophilic fiber that contributes to adhesion of a water-absorbent resin in an absorbent material is reduced due to the recent trend of reducing the thickness of an absorbent material, problems emerge such that the water-absorbent resins move in an absorbent material during the transportation or consumer's application of water-absorbent articles. An absorbent material containing ununiformly distributed water-absorbent resins is likely to cause gel blocking or rupture when absorbing liquid; therefore, its essential absorption properties cannot be exhibited. Accordingly, to produce absorbent materials, a water-absorbent resin requires the following two contradictory properties: excellent impact resistance strength (particle strength) while having an appropriate median particle size, and excellent adhesion to fibers while having excellent powder flowability.

To attain these individual objects, various production techniques of different water-absorbent resins are suggested. Typical methods of producing water-absorbent resin include a reversed suspension polymerization method in which an aqueous monomer solution is suspended in a particle state in a hydrophobic organic solvent (dispersion medium) and then polymerized, and an aqueous solution polymerization method in which an aqueous monomer solution is polymerized without using a dispersion medium .

For example, in the technical field of reversed phase suspension polymerization, there is a method in which acrylate is subjected to a reversed phase suspension polymerization in a petroleum hydrocarbon solvent in the presence of an erythritol fatty acid ester (Patent Literature 3), and there is a method in which an acrylic acid-based monomer is subjected to a reversed phase suspension polymerization using a mixed surfactant of a fatty acid sucrose ester and a fatty acid sorbitan ester, which are solid at an ordinary temperature (Patent Literature 4). Although the spherical water-absorbent resins obtained by the aforementioned reversed phase suspension polymerization methods have excellent powder flowability and particle strength, they do not have a sufficient particle size suitable for producing absorbent materials.

To improve this, spherical particle agglomerates obtained by using the following methods have been suggested. One method comprises subjecting a water-soluble ethylenically unsaturated monomer to a reversed phase suspension polymerization reaction in the first stage, then allowing the water-soluble ethylenically unsaturated monomer to be absorbed in a water-containing gel produced by the polymerization reaction of the first stage, further performing a reversed suspension polymerization reaction, and optionally repeating these operations at least once (Patent Literature 5). Also suggested has been a method comprising polymerizing a water-soluble ethylenically unsaturated monomer by using a water-in-oil type reversed phase suspension polymerization method to form a slurry solution, and adding a polymerizable monomer to perform polymerization, thereby forming agglomerate particles of highly absorbent polymer particles (Patent Literature 6). By using these methods, although water-absorbent resins having excellent flowability and an appropriate particle size can be obtained, they have a disadvantage in that adhesion to fibers is insufficient.

Separately, in the technical field of aqueous solution polymerization, powder flowability and particle strength are insufficient although an appropriate particle size can be obtained by using techniques such as a method of mixing fine water-absorbent resin powders having a smaller particle size with a hydrated gel of water-absorbent resin during polymerization (Patent Literature 7) and a method of surface-cross-linking a mixture of a water-absorbent resin primary particle and a water-absorbent resin granule (Patent Literature 8). To improve this, a method of mixing fine particles other than a water-absorbent resin with a hydrated water-absorbent resin has been suggested (Patent Literature 9); however, the method provides insufficient powder flowabilty and particle strength when compared to the products obtained by using the aforementioned reversed phase suspension polymerization methods.

Some techniques to improve adhesion to fibers have been suggested. Examples of the methods include a method using a non-angular, non-spherical water-absorbent resin having a ratio of average particle length and average particle breadth of 1.5 to 20 (Patent Literature 10), a method in which a water-absorbent resin is obtained by gradually adding a monomer after a precedent reversed phase suspension polymerization to perform a batchwise reversed phase suspension polymerization (Patent Literature 11), and a method using a water-absorbent resin in which granule particles have an aspect ratio (particle length/particle breadth) of 1.5 or more (Patent Literature 12). However, when the ratio of the particle length/particle breadth of a water-absorbent resin is raised and the water-absorbent resin has an elongated shape, the adhesion to fibers may be improved; however, the powder flowability and the particle strength tend to be reduced. Therefore, these methods are not suitable for producing thin absorbent materials.

Hence, development has been desired for a water-absorbent resin having features suitable for producing a thin absorbent material, i.e., having excellent general water absorption properties, an appropriate particle size, and a strong particle strength as well as excellent adhesion to fibers and powder flowability.

Citation List

Patent Literature

PTL 1: Japanese Unexamined Patent Publication No. H9-510889 (Translation of PCT Application)
PTL 2: Japanese Unexamined Patent Publication No. H8-57311
PTL 3: Japanese Unexamined Patent Publication No. H1-294703
PTL 4: Japanese Unexamined Patent Publication No. H2-153907
PTL 5: Japanese Unexamined Patent Publication No. H3-227301
PTL 6: Japanese Unexamined Patent Publication No. H5-17509
PTL 7: Japanese Unexamined Patent Publication No. H5-43610
PTL 8: Japanese Unexamined Patent Publication No. HII-140194
PTL 9: Japanese Unexamined Patent Publication No. 2008-533213
PTL 10: Japanese Unexamined Patent Publication No. H2-196802
PTL 11: Japanese Unexamined Patent Publication No. 2002-284803
PTL 12: Japanese Unexamined Patent Publication No. 2004-2891

EP 2 011 803 A1 discloses a process for the production of water-absorbent resin particles and water-absorbent resin particles produced by the process. In the water-absorbent resin particles a median particle size of primary particles (d) and a median particle size of secondary particles (T) satisfy the relationship of the Formula 5d/3+150<D<5d+150.

EP 2 184 300 A1 relates to a water-absorbing resin suitable for use in sanitary products. The water-absorbent resin is produced by reversed-phase suspension polymerization method which uses a small amount of a petroleum hydrocarbon dispersion medium remaining therein.

EP 2 674 441 A1 discloses a manufacturing method for a water-absorbing resin which method includes a step 1 of dispersing a first aqueous solution containing a partially neutralized product (A) of a water-soluble ethylenically saturated monomer having acid groups in the molecule in the petroleum-type hydrocarbon dispersion medium in the presence of a dispersing agent and then polymerizing the resulting dispersion to obtain a slurry containing primary particles of a polymer, and a step 2 of adding a second aqueous solution containing a partially neutralized product (B) of a water-soluble ethylenically saturated monomer having acid groups in the molecule to the slurry obtained in step 1, and then polymerizing the mixture to obtain a slurry in which the primary particles are agglomerated.

WO2006/014031 A1 discloses a particulate water-absorbing agent comprising water-absorbing resin particles which are obtained by polymerization of an unsaturated monomer containing an acid group and/or a salt thereof and which are at least ones of nearly spherical shapes, aggregates thereof and aggregates derived from nearly spherical shapes, and wherein the water-absorbing agent satisfies further requirements set forth in the document with regard to the centrifuge retention capacity, the mass median particle size, the ratio of particles having a defined diameter and the content of volatile organic compounds.

Summary of Invention

Technical Problem

The main object of the present invention is to provide a water-absorbent resin having a suitable particle size, strong particle strength, excellent adhesion to fibers, and excellent flowability as powders as well as properties suitable for a water-absorbent agent used in a hygienic material, such as excellent general absorption properties as a water-absorbent resin, and to provide a process for producing the same. Another object of the present invention is to provide an absorbent material and an absorbent article using the water-absorbent resin.

Solution to Problem

To achieve the above objects, the present inventors conducted extensive research and as a result found the following. A water-absorbent resin in the form of a secondary particle that satisfies a specific aspect ratio and a particle size, the water-absorbent resin being obtained by agglomerating primary particles that satisfy a specific aspect ratio and a particle size, has strong particle strength, excellent adhesion to fibers, and excellent flowability as powders as well as excellent general absorption properties as a water-absorbent resin.

Specifically, the present invention relates to a water-absorbent resin in the form of a secondary particle which is obtainable by agglomerating primary particles having an aspect ratio of 1.4 to 50 and a median particle size (d) of 50 to 600 $\mu$m, the secondary particle having an aspect ratio of 1.0 to 3.0 and a median particle size (D) of 100 to 2,000 $\mu$m, and to further embodiments set forth in the annexed claims.

Advantageous Effects of Invention

The water-absorbent resin in the form of the secondary particle according to the present invention has an appropriate particle size and strong particle strength as well as excellent general absorption properties. The water-absorbent resin of the present invention has excellent adhesion to fibers as well as excellent flowability as powders due to a small aspect ratio. Therefore, since a thin absorbent material using the water-absorbent resin of the present invention has a high liquid absorption property and a shape retention property, the material can be suitably used in a thin absorbent article.

Description of Embodiments

Water-absorbent resin of the present invention

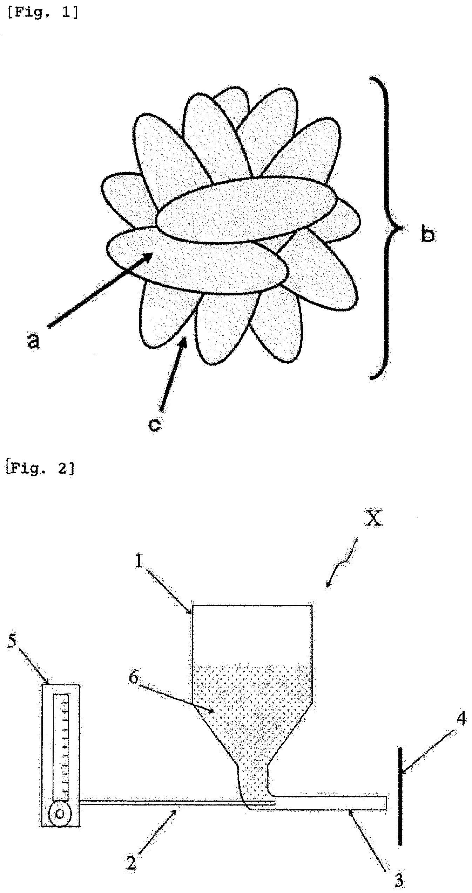

The water-absorbent resin of the present invention is explained demonstrating a schematic view shown in Fig. 1. The water-absorbent resin is in the form of the secondary particle in which primary particles a having a curved surface are agglomerated.

The primary particles have an aspect ratio of 1.4 to 50, and preferably 1.6 to 30.

When the primary particles have an aspect ratio of 1.1 or more, the surface depression c of the secondary particle in which the primary particles are agglomerated becomes deep, which makes it possible to attain excellent adhesion to fibers when the resin is used as an absorbent material together with fiber, etc. When the primary particles have an aspect ratio of 200 or less, the primary particles can be easily agglomerated to form a secondary particle and the contact surfaces between the primary particles are increased. Therefore, the secondary particle after agglomeration has an improved strength as a water-absorbent resin.

The primary particles have a median particle size (d) of 50 to 600 $\mu$m, preferably 60 to 500 $\mu$m, more preferably 80 to 450 $\mu$m, and even more preferably 100 to 400 $\mu$m.

When the primary particles have a median particle size of 50 $\mu$m or more, the secondary particle formed by the agglomeration of the primary particles has a particle size within an appropriate range and can improve the powder flowability as a water-absorbent resin. Further, the secondary particle formed by the agglomeration of the primary particles has a deep surface depression c and can improve the adhesion to fibers when used with fiber in an absorbent material.

When the primary particles have a median particle size of 600 $\mu$m or less, the secondary particle formed by the agglomeration of the primary particles has a particle size within an appropriate range and can provide a good feel when used in an absorbent material. Moreover, since the contact surfaces of the primary particles are increased, the secondary particle formed by the agglomeration of the primary particles has an improved strength as a water-absorbent resin.

The form of the primary particles is not particularly limited; however, the primary particles preferably include a curved surface alone. Specifically, a comma-shaped bead-like shape , an oval-spherical shape, a sausage shape, and a rugby ball shape are preferable. Since the secondary particle is formed by the agglomeration of the primary particles having such a shape, the flowability as powders is improved and the secondary particle after agglomeration is likely to be closely filled. Therefore, the secondary particle is less likely to be broken when subjected to a collision, and a water-absorbent resin having high particle strength can be obtained.

The secondary particle has an aspect ratio of 1.0 to 3.0, preferably 1.0 to 2.5, more preferably 1.0 to 2.0, even more preferably 1.0 to 1.7, and particularly preferably 1.0 to 1.4.

The water-absorbent resin of the secondary particle has a median particle size (D) of 100 to 2,000, preferably 200 to 1,500, more preferably 300 to 1,200, and particularly preferably 360 to 1,000 $\mu$m.

When the secondary particle having a median particle size of 100 $\mu$m or more is used as a water-absorbent resin in an absorbent material, a phenomenon in which liquid diffusion is inhibited, i.e., gel blocking, is not likely to take place, and excellent flowability as powders is attained, without causing adverse effects on the production efficiency of the absorbent material. The secondary particle having a median particle size of 2,000 $\mu$m or less is preferable because it attains excellent feel and flowability when used as a water-absorbent resin in an absorbent material.

Any material can be used as a material of the water-absorbent resin of the present invention as long as it is generally used in a water-absorbent resin. Examples thereof include hydrolysates of starch-acrylonitrile graft copolymers, neutralized products of starch-acrylate graft polymers, saponified products of vinyl acetate-acrylic ester copolymers, and crosslinked polymers of partially neutralized acrylic acid compound. Of these, in view of the production amount, production st, and water-absorbent property, crosslinked polymers of partially neutralized acrylic acid compound are preferable.

The water-absorbent resin of the present invention generally has a particle size distribution uniformity of 1.0 to 2.2, preferably 1.0 to 2.0, and more preferably 1.2 to 1.8. When the water-absorbent resin is used as an absorbent material, it is not preferable to use large particles in a large amount because the absorbent material after compression becomes partially rigid. It is also not preferable to use small particles in a large amount, because the particles are likely to move in a thin absorbent material, and uniformity is impaired. Therefore, the water-absorbent resin used in an absorbent material preferably has a narrow particle size distribution, in other words, a small uniformity degree of particle size distribution. Since the water-absorbent resin of the present invention that satisfies the aforementioned range has a small uniformity degree of particle size distribution, it is suitably used in the absorbent material.

One of the indices that feature the water-absorbent resin of the present invention is water absorption capacity. The water-absorption capacity is generally 30 g/g or more, preferably 35 to 85 g/g, more preferably 40 to 75 g/g, and particularly preferably 45 to 70 g/g. By satisfying such a numerical range, gel can be kept strong to prevent gel blocking, and excess cross-linking can be inhibited to increase the absorption capacity. The water absorption capacity can be measured by using a method described in the Examples shown below.

One of the indices that feature the water-absorbent resin of the present invention is a particle size retaining rate after a particle collision test. The particle size retaining rate is generally 80% or more, preferably 85% or more, and more preferably 90% or more. The higher the particle size retaining rate, the greater the particle strength. The water-absorbent resin of the present invention that satisfies the above numerical range is less likely to be broken when subjected to a collision during the production of an absorbent material and is less likely to increase the fine powder content. Moreover, the quality of the absorbent material is stabilized, which ensures high performance. The particle size retaining rate after a particle collision test can be measured by using a method described in the Examples shown below.

One of the indices that feature the water-absorbent resin of the present invention is a water absorption capacity of saline solution under load. The water absorption capacity of saline solution under load is generally 12 mL/g or more, preferably 14 mL/g or more, more preferably 16 mL/g or more, and particularly preferably 18 mL/g or more. The water-absorbent resin having a higher water absorption capacity of saline solution under load can absorb more liquid when used as an absorbent material under load. The water-absorbent resin of the present invention that satisfies the aforementioned numerical range can maintain the properties required when used in an absorbent material. The water absorption capacity of saline solution under load of the water-absorbent resin under load can be measured by using a method described in the Examples shown below.

One of the indices that feature the water-absorbent resin of the present invention is the physiological saline solution absorption retaining rate under load obtained after a particle collision test. The physiological saline solution absorption retaining rate is generally 80% or more, preferably 85% or more, and more preferably 90% or more. The water-absorbent resin having a high physiological saline solution absorption retaining rate has a high absorption capacity under load even when subjected to a collision during the production of an absorbent material; therefore, the properties used as an absorbent material can be maintained. The water-absorbent resin of the present invention that satisfies the aforementioned numerical range can maintain the properties used as an absorbent material. The physiological saline solution absorption retaining rate under load after a particle collision test can be measured according to a method described in the Examples shown below.

One of the indices that feature the water-absorbent resin of the present invention is an index of adhesion to fibers. The index of adhesion to fibers is the value defined by the ratio of the water-absorbent resin that can maintain the fiber adhesion property after shaking obtained when the water-absorbent resin is used in an absorbent material. The water-absorbent resin of the present invention generally has an index of adhesion to fibers of 50 to 100, preferably 60 to 100, more preferably 70 to 100, and particularly preferably 80 to 100. The water-absorbent resin having a high index of adhesion to fibers is less likely to cause variations when used in an absorbent material. The water-absorbent resin of the present invention that satisfies the aforementioned numerical range can maintain the liquid absorption property when used in an absorbent article. The index of adhesion to fibers of the water-absorbent resin can be measured by using a method described in the Examples shown below.

One of the indices that feature the water-absorbent resin of the present invention is the flow index of powders. The flow index is defined by the ratio of salt flow amount in a powder transfer device. The water-absorbent resin of the present invention generally has a flow index of 70 to 200, preferably 80 to 180, and more preferably 90 to 160. When the water-absorbent resin has a high flow index, powders can be easily transferred and the supply amount can be stabilized in the production of absorbent material. Accordingly, variations in the amount of the water-absorbent resin in the absorbent material are reduced and transfer problems due to the bridge formation in a powder hopper are less likely to occur. The water-absorbent resin of the present invention that satisfies the aforementioned numerical range exhibits advantageous effects in the production of an absorbent material. The powder flow index of the water-absorbent resin can be measured by using a method described in the Examples shown below.

Method of producing the water-absorbent resin of the present invention

There is no limitation on the method for producing primary particles that form the water-absorbent resin in the form of the secondary particle according to the present invention. For example, a water-absorbent resin that is in a water-containing gel state has an appropriate particle size and a uniform particle degree and is obtained by a first stage of polymerization according to a reversed phase suspension polymerization method, or a dried resin thereof; a water-absorbent resin that has a wide particle size distribution and is obtained by drying and pulverizing an agglomerated water-containing gel obtained by using an aqueous solution polymerization method can be used, or a classified resin thereof can be used. The primary particles are preferably obtained by using a reversed phase suspension polymerization method from the viewpoint of less burden such as for pulverization and sieving ; production process ease; and excellent properties of the resulting water-absorbent resin, such as a water-absorption property and particle strength.

The primary particles obtained by using the aforementioned method are agglomerated to form a secondary particle, thereby obtaining the water-absorbent resin of the present invention. The agglomeration method is hot particularly limited, and examples thereof include a method in which the primary particles obtained by using the above method are granulized and agglomerated using a binder, such as water or an adhesive; also included is a multistage reversed phase suspension polymerization method in which the primary particles are formed by using a reversed phase suspension polymerization method, and then the primary particles are agglomerated by using a reversed phase suspension polymerization method. Of these, the latter method, i.e., multistage reversed phase suspension polymerization, is preferable from the viewpoint of production process ease, excellent water-absorption property, particle strength, and other properties of the resulting water-absorbent resin.

The present invention is explained below by demonstrating a method that uses a multistage reversed phase suspension polymerization method including the following steps 1 and 2 as an embodiment of the method for producing the water-absorbent resin in the form of the secondary particle according to the present invention. However, the present invention is not limited to this embodiment.

(1) step 1, in which a water-soluble ethylenically unsaturated monomer is subjected to a polymerization reaction in the presence of a thickener and a dispersion stabilizer to form a slurry in which primary particles having an aspect ratio of 1.4 to 50 are dispersed.

(2) step 2, in which the slurry obtained in step 1 is cooled to precipitate the dispersion stabilizer, and a water-soluble ethylenically unsaturated monomer is added thereto to perform a polymerization reaction, thereby agglomerating primary particles dispersed in the slurry to form a water-absorbent resin in the form of the secondary particle.

step 1

The reversed phase suspension polymerization is a method in which an aqueous monomer solution is stirred in a dispersion medium in the presence of a dispersion stabilizer to conduct suspension, and the monomer is polymerized in a droplet aqueous solution. Specifically, in step 1, a water-soluble ethylenically unsaturated monomer is subjected to a polymerization reaction in the presence of a thickener and a dispersion stabilizer to form a slurry in which a polymer in the form of the primary particle that satisfies the aforementioned particle size and aspect ratio is dispersed.

The thickener is at least one member selected from the group consisting of hydroxyethyl cellulose, hydroxypropyl cellulose, methyl cellulose, carboxymethyl cellulose, polyacrylic acid, (partially) neutralized products of polyacrylic acid, polyethylene glycol, polyacrylamide, polyethylene imine, dextrin, sodium alginate, polyvinyl alcohol, polyvinyl pyrrolidone, and polyethylene oxide. Of these, hydroxyethyl cellulose, carboxymethyl cellulose, polyvinyl alcohol, and polyvinyl pyrrolidone are preferable from the viewpoint of high solubility in the aqueous monomer solution and high-viscosity expression effects.

In general, in the reversed suspension polymerization reaction, when the rotational speeds of stirring are the same, the higher the viscosity of the aqueous monomer solution, the larger the particle size of the resulting primary particles. Further, even when the primary particle sizes are the same, the particle having a high aqueous monomer solution viscosity and a high stirring speed has a larger aspect ratio.

Although the amount of the thickener cannot be determined, because the viscosity of the aqueous monomer solution depends on the kind of thickener, the thickener is generally added in an amount such that the viscosity of the aqueous monomer solution is 10 to 500,000 mPa/s, preferably 20 to 300,000 mPa/s, and more preferably 50 to 100,000 mPa/s (Brookfield viscosity, 20°C, 6 rpm). The amount of the thickener relative to the aqueous monomer solution is generally 0.005 to 10% by mass, preferably 0.01 to 5% by mass, and more preferably 0.03 to 3% by mass. By adding the aforementioned amount of the thickener to the aqueous monomer solution, the resulting primary particles have an aspect ratio in the aforementioned range.

As a dispersion medium, a petroleum hydrocarbon dispersion medium can be used. Examples of the petroleum hydrocarbon dispersion medium include aliphatic hydrocarbons, such as n-hexane, n-heptane, n-octane, and ligroin; alicyclic hydrocarbons, such as cyclopentane, methylcyclopentane, cyclohexane, and methylcyclohexane; and aromatic hydrocarbons, such as benzene, toluene, and xylene. Of these, n-hexane, n-heptane, and cyclohexane are preferably used because they are easily available industrially, stable in quality, and inexpensive. These dispersion media can be used alone or in a combination of two or more.

Examples of the water-soluble ethylenically unsaturated monomer used include (meth)acrylic acid (herein "acryl" and "methacryl" collectively refer to "(meth)acryl," and "acrylate" and "methacrylate" collectively refer to "(meth)acrylate", 2-(meth)acrylamide-2-methylpropanesulfonic acid, and salts thereof; nonionic monomers, such as (meth)acrylamide, N,N-dimethyl(meth)acrylamide, 2-hydroxyethyl(meth)acrylate, N-methylol(meth)acrylamide, and polyethylene glycol mono(meth)acrylate; amino-group-containing unsaturated monomers, such as N,N-diethylaminoethyl (meth)acrylate, N,N-diethylaminopropyl(meth) acrylate, diethylaminopropyl(meth)acrylamide, and quaternary compounds thereof. At least one member selected from these groups can be used. Of these, acrylic acid, methacrylic acid, and salts thereof, acrylamide, methacrylamide, and N,N-dimethylacrylamide are preferably used.

The concentration of the water-soluble ethylenically unsaturated monomer in the aqueous monomer solution is not particularly limited. It is preferable that the concentration of the monomer in the aqueous monomer solution be 20% by mass to a saturated concentration, more preferably 30 to 55% by mass, and even more preferably 35 to 46% by mass. By setting the concentration of the water-soluble ethylenically unsaturated monomer in this range, high production efficiency can be maintained while avoiding excess reaction.

When a monomer containing an acid group such as (meth)acrylic acid or 2-(meth)acrylamide-2-methylpropanesulfonic acid is used as the water-soluble ethylenically unsaturated monomer, the acid group may be neutralized in advance with an alkaline neutralizing agent. Examples of the alkaline neutralizing agent include alkali metal compounds such as sodium hydroxide and potassium hydroxide, and ammonia. The alkaline neutralizing agent may be in the form of aqueous solution. These alkaline neutralizing agents can be used alone or in a combination of two or more.

The degree of neutralization of all the acid groups with the alkaline neutralizing agent is generally 0 to 100% by mol, preferably 30 to 90% by mol, and more preferably 50 to 80% by mol from the viewpoint of increasing osmotic pressure of the resulting water-absorbent resin in the form of the secondary particle to increase the absorption property, and not causing any disadvantages in safety due to the presence of an excess alkaline neutralizing agent.

As a dispersion stabilizer, a surfactant and a polymeric dispersion agent are used.

The surfactant is at least one member selected from the group consisting of sucrose fatty acid esters, polyglycerol fatty acid esters, sorbitan fatty acid esters, polyoxyethylene sorbitan fatty acid esters, polyoxyethylene glycerol fatty acid esters, sorbitol fatty acid esters, polyoxyethylene sorbitol fatty acid esters, polyoxyethylene alkyl ethers, polyoxyethylene alkylphenyl ethers, polyoxyethylene castor oil, polyoxyethylene cured castor oil, alkylallylformaldehyde condensed polyoxyethylene ethers, polyoxyethylene polyoxypropylene block copolymers, polyoxyethylene polyoxypropyl alkyl ethers, polyethylene glycol fatty acid esters, alkyl glucosides, N-alkyl glucone amides, polyoxyethylene fatty acid amides, polyoxyethylene alkylamines, phosphoric esters of polyoxyethylene alkyl ethers, and phosphoric esters of polyoxyethylene alkylallyl ethers.

Of these, from the viewpoint of dispersion stability of an aqueous monomer solution, sucrose fatty acid esters, polyglyceryl fatty acid esters, and sorbitan fatty acid esters are preferably used. These dispersion stabilizers can be used alone or in a combination of two or more.

The HLB of a surfactant used as a dispersion stabilizer cannot be generalized, because the shape of the primary particles depends on the kind of surfactant. For example, in the case of sucrose fatty acid esters or sorbitan fatty acid esters, a surfactant having an HLB of 5 or less can be used; in the case of polyglyceryl fatty acid esters, a surfactant having an HLB of 10 or less can be used.

Together with a surfactant, a polymeric dispersion agent is used as a dispersion stabilizer. The polymeric dispersion agent is at least one member selected from the group consisting of maleic anhydride-modified polyethylene, maleic anhydride-modified polypropylene, maleic anhydride-modified ethylene-propylene copolymers, maleic anhydride-modified EPDM (ethylene-propylene-diene terpolymer), maleic anhydride-modified polybutadiene, ethylene-maleic anhydride copolymers, ethylene-propylene-maleic anhydride copolymers, butadiene-maleic anhydride copolymers, oxidized polyethylene, ethylene-acrylic acid copolymers, ethyl cellulose, and ethyl hydroxyethyl cellulose. Of these, maleic anhydride-modified polyethylene, maleic anhydride-modified polypropylene, maleic anhydride-modified ethylene-propylene copolymers, oxidized polyethylene, and ethylene-acrylic acid copolymers are preferably used from the viewpoint of dispersion stability of an aqueous monomer solution. These polymeric dispersion agents can be used alone or in a combination of two or more.

The dispersion stabilizer is generally used in an amount of 0.1 to 5 parts by mass, and preferably 0.2 to 3 parts by mass based on 100 parts by mass of the aqueous monomer solution to keep an excellent dispersion state of the aqueous monomer solution in the petroleum hydrocarbon dispersion medium, which is used as a dispersion medium, and to obtain a dispersion effect that corresponds to the amount used.

The aqueous monomer solution may contain a radical polymerization initiator. Examples of the radical polymerization initiator include persulfates, such as potassium persulfate, ammonium persulfate, and sodium persulfate; peroxides such as, methyl ethyl ketone peroxide, methyl isobutyl ketone peroxide, di-t-butyl peroxide, t-butyl cumyl peroxide, t-butyl peroxyacetate, t-butyl peroxyisobutylate, t-butyl peroxypivalate, and hydrogen peroxide; and azo compounds, such as 2,2'-Azobis(2-methylpropionamidine)dihydrochloride, 2,2'-azobis[2-(N-phenylamidino)propane]dihydrochloride, 2,2'-azobis[2-(N-allylamidino)propane]dihydrochloride, 2,2'-azobis{2-[1-(2-hydroxyethyl)-2-imidazoline-2-yl]propane}dihydrochloride, 2,2'-azobis (2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). These radical polymerization initiators can be used alone or in a combination of two or more.

The radical polymerization initiator is generally used in an amount of 0.005 to 1% by mol, based on the total amount of the monomer used in this step. By setting the amount of the radical polymerization initiator in this range, no abrupt polymerization occurs and a monomer polymerization reaction does not require a long period of time, a good reversed suspension polymerization reaction is performed.

The radical polymerization initiator can be also used as a redox polymerization initiator together with a reducing agent, such as sodium sulfite, sodium hydrogen sulfite, ferrous sulfate, and L-ascorbic acid.

To control the absorption property of the resulting water-absorbent resin in the form of the secondary particle, a chain transfer agent may be added to the aqueous monomer solution. Examples of the chain transfer agent include hypophosphites, thiols, thiolic acids, secondary alcohols, and amines.

A crosslinking agent (internal crosslinking agent) may be added, if necessary, to the aqueous monomer solution to carry out polymerization. As the internal crosslinking agent, compounds having two or more polymerizable unsaturated groups can be used. Examples of the crosslinking agent include di- or tri-(meth)acrylate esters of polyols such as (poly)ethylene glycol (for example, "polyethylene glycol" and "ethylene glycol" as used herein are collectively refer to "(poly)ethylene glycol"), (poly)propylene glycol, trimethylolpropane, glycerol polyoxyethylene glycol, polyoxypropylene glycol, and (poly)glycerol; unsaturated polyesters obtained by reacting the above-mentioned polyol with an unsaturated acid, such as maleic acid and fumaric acid; bisacrylamides, such as N,N'-methylenebis(meth)acrylamide; di- or tri(meth)acrylate esters obtained by reacting a polyepoxide with (meth)acrylic acid; carbamyl esters of di(meth)acrylic acid obtained by reacting a polyisocyanate, such as tolylenediisocyanate or hexamethylenediisocyanate, with hydroxyethyl (meth) acrylate; allylated starch; allylated cellulose; diallyl phthalate; N,N',N"-triallyl isocyanurate; and divinylbenzene.

As the internal crosslinking agent, in addition to the aforementioned compounds having two or more polymerizable unsaturated groups, compounds having two or more other reactive functional groups can be used. Examples thereof include glycidyl-group-containing compounds, such as (poly)ethylene glycol diglycidyl ethers, (poly)propylene glycol diglycidyl ethers, and (poly)glycerol diglycidyl ethers; (poly)ethylene glycol; (poly)propylene glycol; (poly)glycerol; pentaerythritol; ethylenediamine; polyethyleneimine; and glycidyl(meth) acrylate. These internal crosslinking agents can be used alone or in a combination of two or more.

The amount of the internal crosslinking agent added is generally 1% by mol or less, preferably 0.5% by mol or less, and more preferably 0.001 to 0.25% by mol based on the total amount of the monomer used in this step from the viewpoint of sufficiently enhancing the absorption property of the resulting water-absorbent resin.

The reaction temperature of the polymerization reaction in this step varies depending on the presence or absence of a radical polymerization initiator and the kind of a radical polymerization initiator used. The reaction temperature is generally 20 to 110°C, and preferably 40 to 90°C. By performing the monomer polymerization reaction at a temperature in this range, the monomer polymerization reaction can be terminated within an appropriate time. In addition, since heat generated in the polymerization can be easily removed, the polymerization reaction proceeds smoothly. The reaction time is generally 0.1 to 4 hours.

The particle size of the primary particles produced in this step can be adjusted, for example, by changing the rotational speed of stirring during polymerization reaction using various kinds of stirring impellers. As the stirring impellers, a propeller impeller, a paddle impeller, an anchor impeller, a turbine impeller, a Phaudler impeller, a ribbon impeller, a Fullzone impeller (produced by Shinko Pantec Co., Ltd.), a MAXBLEND impeller (produced by Sumitomo Heavy Industries, Ltd.), and a Super-Mix impeller (produced by Satake Chemical Equipment Mfg., Ltd.) can be used. In general, when the same kinds of stirring impellers are used, the higher the rotational speed of stirring, the smaller the particle size of the primary particles.

Step 2

Step 2 is a step wherein the slurry having the polymer in the form of the primary particles dispersed therein, which is obtained in step 1, is cooled to precipitate the dispersion stabilizer; and subsequently, a water-soluble ethylenically unsaturated monomer is added to the slurry to carry out a polymerization reaction, thereby agglomerating the polymer in the form of the primary particles dispersed in the slurry so as to produce a water-absorbent resin in the form of the secondary particle in which the above-described particle size and aspect ratio are satisfied.

Although the cooling temperature is not particularly limited, as the precipitation temperature varies depending on the type of dispersion stabilizer and dispersion medium used in step 1, it is usually 10 to 50° C, and preferably 20 to 40° C. The precipitation of the dispersion stabilizer can be confirmed by the presence of white turbidity in the slurry. Specifically, such white turbidity can be determined by visual observation or by the use of a turbidity meter. It is also possible to control the particle size and form of the water-absorbent resin by changing the precipitation temperature. Specifically, a water-absorbent resin having a small median particle size can be produced at a temperature of about 50°C, and a water-absorbent resin having a large median particle size can be produced at a temperature of about 10°C.

Although the water-soluble ethylenically unsaturated monomer to be added to the slurry is not particularly limited, it can be suitably selected from the monomers included in the examples of water-soluble ethylenically unsaturated monomers used in step 1. In particular, it is preferable to use the same compound as the monomer used in step 1, from the viewpoint that an appropriate particle size and a narrow particle size distribution can be easily obtained.

The water-soluble ethylenically unsaturated monomer is usually used in an amount of 90 to 200 parts by mass, preferably 110 to 180 parts by mass, more preferably 120 to 160 parts by mass, relative to 100 parts by mass of the water-soluble ethylenically unsaturated monomer used in step 1. When the water-soluble ethylenically unsaturated monomer is used in an amount of 90 parts by mass or more, it provides a sufficient amount of monomer to agglomerate the polymer in the form of the primary particles. Accordingly, an optimum agglomeration of the polymer is formed, and the resulting water-absorbent resin in the form of the secondary particle can be formed with a median particle size in the above-mentioned range. The water-absorbent resin having increased particle strength can be obtained.

Further, when the water-soluble ethylenically unsaturated monomer is used in an amount of 200 parts by mass or less, it is possible to prevent the depressions in the surface of the water-absorbent resin from being filled with fine particles formed by a polymerization of excess monomer. Therefore, when the resulting water-absorbent resin is used as an absorbent material, it is possible to prevent the occurrence of gel blocking by the fine particles.

Any compound included in the examples of radical polymerization initiators in step 1 may be used as the radical polymerization initiator to be added to the monomer in step 2. It is preferable to use the same compound used in step 1. Further, the radical polymerization initiator is usually used in an amount of about 0.005 to 1% by mol relative to the total amount of the monomer used in step 2.

Note that the radical polymerization initiator can be used as a redox polymerization initiator in combination with a reducing agent such as sodium sulfite, sodium hydrogen sulfite, ferrous sulfate, and L-ascorbic acid.

In step 2, a chain transfer agent may be used together with the water-soluble ethylenically unsaturated monomer in order to control the absorption property of the water-absorbent resin in the form of the secondary particle. The chain transfer agent to be used may be any compound included in the examples of chain transfer agents in step 1.

Although the reaction temperature of the polymerization reaction in step 2 varies depending on the radical polymerization initiator used, it is preferably in the same temperature range as in step 1.

A step of post-crosslinking the water-absorbent resin in the form of the secondary particle using a cross-linking agent may be performed after step 2. The timing for adding the post-crosslinking agent is not particularly limited, and the post-crosslinking agent is added in the presence of usually 1 to 400 parts by mass of water, preferably 5 to 200 parts by mass of water, more preferably 10 to 100 parts by mass of water, and particularly preferably 20 to 60 parts by mass of water, relative to 100 parts by mass of the total amount of the water-soluble ethylenically unsaturated monomer used. As described above, the timing for adding the post-crosslinking agent is selected in accordance with the amount of water contained in the water-absorbent resin. As a result, the water-absorbent resin is more suitably subjected to crosslinking on its surface or near its surface, thereby producing a water-absorbent resin having an excellent water absorption capacity of saline solution under load.

The cross-linking agent used for post-crosslinking is not particularly limited insofar as it is a compound having two or more reactive functional groups. Specific examples thereof include diglycidyl group-containing compounds such as (poly)ethylene glycol diglycidyl ether, (poly)glycerol (poly)glycidyl ether, (poly)propylene glycol diglycidyl ether, and (poly)glycerol diglycidyl ether; (poly)ethylene glycol; (poly)propylene glycol; (poly)glycerol; pentaerythritol; ethylenediamine; and polyethyleneimine. Of these, (poly)ethylene glycol diglycidyl ether, (poly)propylene glycol diglycidyl ether, and (poly)glycerol diglycidyl ether are particularly preferable. These crosslinking agents may be used singly, or in a combination of two or more.

The post-crosslinking agent is usually used in an amount from 0.005 to 1% by mol, preferably from 0.01% to 0.75 by mol, more preferably from 0.02 to 0.5% by mol relative to the total amount of the monomer used for the polymerization reaction, from the viewpoint of not lowering the absorption properties of the resulting water-absorbent resin in the form of the secondary particle; and increasing the crosslinking density on its surface or near its surface so as to enhance the water absorption capacity of saline solution under load.

When the post-crosslinking agent is added, the post-crosslinking agent may be added as it is, or added in a form of aqueous solution. If necessary, the hydrophilic organic solvent may be used as a solvent. Examples of hydrophilic organic solvents include lower alcohols such as methyl alcohol, ethyl alcohol, n-propyl alcohol and isopropyl alcohol; ketones such as acetone and methyl ethyl ketone; ethers such as diethyl ether, dioxane, and tetrahydrofuran; amides such as N,N-dimethylformamide; and sulfoxides such as dimethyl sulfoxide like. These hydrophilic organic solvents may be used singly, or in a combination of two or more. Alternatively, these hydrophilic organic solvents may be used as a mixed solvent with water.

The reaction temperature in the post-crosslinking treatment is preferably 50 to 250°C, more preferably 60 to 180°C, further preferably 60 to 140°C, and still further preferably 70 to 120°C. The reaction time is usually from 1 hour to 5 hours.

The water-absorbent resin in the form of the secondary particle obtained after treatment with a cross-linking agent may further be subjected to a drying treatment after step 2, or as the need arises. The final water content in the water-absorbent resin is usually 20% or less, preferably 2 to 15%, more preferably 5 to 10%, from a viewpoint of achieving good flowability of the water-absorbent resin as powder. The drying treatment may be carried out under normal pressure or under a nitrogen stream in order to increase the drying efficiency. When the drying is carried out under normal pressure, the drying temperature is preferably 70 to 250°C, more preferably 80 to 180°C, further preferably 80 to 140°C, and still further preferably 90 to 130°C. Further, when the drying is carried out under reduced pressure, the drying temperature is preferably 60 to 100°C, more preferably 70 to 90°C.

The water-absorbent resin in the form of the secondary particle produced by the above-described steps is suitably used for absorbent materials and absorbent articles that use the same because of the following points: general water absorption properties of the water-absorbent resin, such as water absorption capacity and water absorption capacity of saline solution under load., are excellent; the water-absorbent resin has strong particle strength while having a moderate particle size, and has excellent adhesion to fibers; and the water-absorbent resin has excellent powder flowability because of the small aspect ratio.

Absorbent Material of the Present Invention

The water-absorbent resin of the present invention has properties that allow the water-absorbent resin to also be effectively used as the absorbent material as described above. The water-absorbent resin can be provided as an absorbent material when used with hydrophilic fibers.

Examples of hydrophilic fibers include cellulose fibers, and artificial cellulose fibers. Note that the hydrophilic fibers may include hydrophobic synthetic fibers insofar as the object of the present invention is not hindered.

The content of water-absorbent resin in the absorbent material is usually about 40 mass% or more, preferably 50 mass% or more, more preferably 60 mass% or more, particularly preferably 70 mass% or more, from the viewpoint of sufficiently absorbing a body fluid such as urine, and providing a comfortable feeling of wear to the user. Further, the content of water-absorbent resin in the absorbent material is usually about 98 mass% or lower, preferably 95 mass% or lower, more preferably 90 mass% or lower, in view of including an appropriate amount of hydrophilic fibers in order to increase the shape retention property of the resulting absorbent material.

Examples of preferable embodiments of the absorbent material include a mixed dispersant obtained by mixing a water-absorbent resin composition and hydrophilic fibers to form a homogeneous composition; and a sandwich structure in which water-absorbent resin is sandwiched between two layers of hydrophilic fibers .

An absorbent article can be formed by including the absorbent material between, for example, a liquid-permeable sheet and a liquid-impermeable sheet.

Examples of liquid-permeable sheets include nonwoven fabrics such as air-through-type, spun bond-type, chemical bond-type, and needle punch-type, which are made of fibers such as polyethylene, polypropylene, and polyester.

Examples of liquid-impermeable sheets include synthetic resin films made of a resin such as polyethylene, polypropylene, and polyvinyl chloride.

The type of absorbent articles is not particularly limited. Representative examples of absorbent articles include hygienic materials such as disposal diapers, sanitary napkins, and incontinence pads; urine-absorbing materials for pets; materials for city engineering and construction such as packing materials; food freshness preservers such as drip absorbents and refrigerants; agricultural and horticultural materials such as water-retaining materials for soil; and water-retaining materials for cables .

Examples

The present invention is described in further detail below based on Examples. However, the present invention is not limited to these Examples.

Example 1

A cylindrical round-bottomed separable flask having an internal diameter of 100 mm, equipped with a reflux condenser, a dropping funnel, a nitrogen gas inlet tube, and a stirring impeller having 2 sets of four pitched paddle impellers (a impeller diameter of 50 mm) as a stirrer was provided. This flask was charged with 500 ml of n-heptane, and 0.92 g of a sucrose stearate having an HLB of 3 (produced by Mitsubishi-Kagaku Foods Corporation, Ryoto sugar ester S-370) and 0.92 g of a maleic anhydride-modified ethylene-propylene copolymer (produced by Mitsui Chemicals, Inc., Hi-wax 1105A) were added thereto. The temperature was raised to 80°C to dissolve the surfactant, and thereafter the solution was cooled to 50°C.

Separately, an Erlenmeyer flask (500 ml capacity) was charged with 92 g of acrylic acid aqueous solution (80.5 mass%), and 167.7 g of sodium hydroxide aqueous solution (18.4 mass%) was added dropwise thereto under external cooling to neutralize 75% by mol. Thereafter, 2.30 g of hydroxyethyl cellulose (produced by Daicel Chemical Industries, Ltd., product number: SP-600) was added and completely dissolved by stirring at room temperature. 0.11 g of potassium persulfate and 9.2 mg of N,N'-methylenebisacrylamide were added and dissolved, thereby preparing an aqueous monomer solution for the first stage. (This aqueous solution had a viscosity of 10,000 mPa.s.)

Setting the rotation speed of the stirrer at 600 rpm, the aqueous monomer solution was added to the separable flask. The flask was maintained at 35°C for 30 minutes while replacing the inside of the system with nitrogen. Subsequently, the flask was immersed in a water bath at 70°C to raise the temperature, and the polymerization was carried out, thereby obtaining polymerized slurry for the first stage. (Note that when the polymerized slurry of this stage was subjected to azeotropic distillation of water and n-heptane using an oil bath at 120°C to distill off only water to the outside of the system and was subsequently dried through n-heptane evaporation, the resulting oval-spherical primary particles had a median particle size of 190 $\mu$m, and an aspect ratio of 1.9.)

Separately, another 500-mL Erlenmeyer flask was charged with 110.4 g of acrylic acid aqueous solution (80.5 mass%), and 149.3 g of sodium hydroxide aqueous solution (24.7 mass%) was added dropwise thereto under external cooling to neutralize 75% by mol. Thereafter, 0.13 g of potassium persulfate and 11.0 mg of N,N'-methylenebisacrylamide were added thereto to and dissolved, thereby preparing an aqueous monomer solution for the second stage. The temperature was maintained at about 24°C.

After the rotation speed for stirring the polymerized slurry was changed to 1,000 r/min, the slurry was cooled to 24°C, and the aqueous monomer solution for the second stage was added into the system. After the inside of the system was maintained for 30 minutes while replacing with nitrogen, the flask was again immersed in a water bath at 70°C to raise the temperature; and the polymerization was carried out, thereby obtaining polymerized slurry for the second stage.

Next, the temperature was raised using an oil bath at 120°C, and the slurry was subjected to azeotropic distillation of water and n-heptane to distill off 259.8 g of water to the outside of the system while refluxing n-heptane. Thereafter, 5.06 g of a 2% aqueous solution of ethylene glycol diglycidyl ether was added thereto, and the mixture was maintained at 80°C for 2 hours. Subsequently, n-heptane was evaporated, and the mixture was dried, thereby obtaining 212.5 g of water-absorbent resin in the form of the secondary particle in which the oval-spherical primary particles are agglomerated. The obtained water-absorbent resin had a median particle size of 600 $\mu$m and a water content of 6%. Table 1 shows the measurement results of each property.

Example 2

The same procedure as in Example 1 was repeated except that the stirring rotation speed for polymerization for the first stage was changed to 400 rpm, and the amount of 2% aqueous solution of ethylene glycol diglycidyl ether to be added after azeotropic dehydration was changed to 11.13 g, thereby obtaining 213.1 g of water-absorbent resin in the form of the secondary particle in which the oval-spherical primary particles are agglomerated. (Note that when the polymerized slurry of this stage was subjected to azeotropic distillation of water and n-heptane using an oil bath at 120°C to distill off only water to the outside of the system and was subsequently dried through n-heptane evaporation, the resulting oval-spherical primary particles had a median particle size of 280 $\mu$m, and an aspect ratio of 1.4.) The obtained water-absorbent resin had a median particle size of 720 $\mu$m and a water content of 7%. Table 1 shows the measurement results of each property.

Example 3

The same procedure as in Example 1 was repeated except that the stirring rotation speed for polymerization for the first stage was changed to 700 rpm, and the amount of 2% aqueous solution of ethylene glycol diglycidyl ether to be added after azeotropic dehydration was changed to 6.07 g, thereby obtaining 214.0 g of water-absorbent resin in the form of the secondary particle in which the oval-spherical primary particles are agglomerated. (Note that when the polymerized slurry of this stage was subjected to azeotropic distillation of water and n-heptane using an oil bath at 120°C to distill off only water to the outside of the system and was subsequently dried through n-heptane evaporation, the resulting oval-spherical primary particles had a median particle size of 110 $\mu$m, and an aspect ratio of 1.6.) The obtained water-absorbent resin had a median particle size of 470 $\mu$m and a water content of 7%. Table 1 shows the measurement results of each property.

Example 4

A cylindrical round-bottomed separable flask having an internal diameter of 100 mm, equipped with a reflux condenser, a dropping funnel, a nitrogen gas inlet tube, and a stirring impeller having 2 sets of four pitched paddle impellers (a impeller diameter of 50 mm) as a stirrer was provided. This flask was charged with 500 ml of n-heptane, and 0.92 g of a sucrose stearate having an HLB of 3 (produced by Mitsubishi-Kagaku Foods Corporation, Ryoto sugar ester S-370) and 0.92 g of a maleic anhydride-modified ethylene-propylene copolymer (produced by Mitsui Chemicals, Inc., Hi-wax 1105A) were added thereto. The temperature was raised to 80°C to dissolve the surfactant, and thereafter the inside of the system was replaced with nitrogen at 78° C.

Separately, a 500 mL-Erlenmeyer flask was charged with 92 g of acrylic acid aqueous solution (80.5 mass%), and 154.1 g of sodium hydroxide aqueous solution (20.0 mass%) was added dropwise thereto under external cooling to neutralize 75% by mol. Thereafter, 2.76 g of hydroxyethyl cellulose (produced by Daicel Chemical Industries, Ltd., product number: SP-600) was added and completely dissolved by stirring at room temperature. 0.11 g of potassium persulfate and 9.2 mg of ethylene glycol diglycidyl ether were added and dissolved, thereby preparing an aqueous monomer solution for the first stage. This aqueous solution had a viscosity of 26,000 mPa·s.

Setting the rotation speed of the stirrer at 600 rpm, the aqueous monomer solution was added at a rate of 5 mL/min while continuing the nitrogen replacement in the inside of the separable flask system to carry out polymerization at 74 to 78°C, thereby obtaining polymerized slurry for the first stage. (Note that when the polymerized slurry of this stage was subjected to azeotropic distillation of water and n-heptane using an oil bath at 120°C to distill off only water to the outside of the system and was subsequently dried through n-heptane evaporation, the resulting comma-shaped bead-like primary particles had a median particle size of 430 $\mu$m, and an aspect ratio of 5.5.)

Separately, another 500 mL-Erlenmeyer flask was charged with 110.4 g of acrylic acid aqueous solution (80.5 mass%), and 149.9 g of sodium hydroxide aqueous solution (24.7 mass%) was added dropwise thereto under external cooling to neutralize 75% by mol. Thereafter, 0.13 g of potassium persulfate and 11.0 mg of ethylene glycol diglycidyl ether were added thereto and dissolved, thereby preparing an aqueous monomer solution for the second stage. The temperature was maintained at about 25°C.

After the rotation speed for stirring the polymerized slurry was changed to 1,000 r/min, the slurry was cooled to 25°C, and the aqueous monomer solution for the second stage was added into the system. After the inside of the system was maintained for 30 minutes while replacing with nitrogen, the flask was immersed in a water bath at 70°C to raise the temperature, and the polymerization was carried out, thereby obtaining polymerized slurry for the second stage.

Next, the temperature was raised using an oil bath at 120°C, and the slurry was subjected to azeotropic distillation of water and n-heptane to distill off 259.8 g of water to the outside of the system while refluxing n-heptane. Thereafter, 4.05 g of a 2% aqueous solution of ethylene glycol diglycidyl ether was added thereto, and the mixture was maintained at 80°C for 2 hours. Subsequently, n-heptane was evaporated, and the mixture was dried, thereby obtaining 213.3 g of water-absorbent resin in the form of the secondary particle in which the comma-shaped bead-like primary particles are agglomerated. The water-absorbent resin had a median particle size of 900 $\mu$m and a water content of 6%. Table 1 shows the measurement results of each property.

Example 5

The same procedure as in Example 4 was repeated except that the stirring rotation speed for polymerization for the first stage was changed to 1,200 rpm, the temperature of the aqueous monomer solution for the second stage and the temperature of the polymerized slurry for the first stage were both changed to 23°C, and the amount of 2% aqueous solution of ethylene glycol diglycidyl ether to be added after azeotropic dehydration was changed to 8.10 g, thereby obtaining 212.8 g of water-absorbent resin in the form of the secondary particle in which the oval-spherical primary particles are agglomerated. (Note that when the polymerized slurry of this stage was subjected to azeotropic distillation of water and n-heptane using an oil bath at 120°C to distill off only water to the outside of the system and was subsequently dried through n-heptane evaporation, the resulting oval-spherical primary particles had a median particle size of 80 $\mu$m, and an aspect ratio of 2.2.) The obtained water-absorbent resin had a median particle size of 390 $\mu$m and a water content of 5%. Table 1 shows the measurement results of each property.

Comparative Example 1

The same procedure as in Example 1 was repeated except that hydroxyethyl cellulose was not added, thereby obtaining 213.9 g of the water-absorbent resin in the form of the secondary particle in which the completely spherical primary particles are agglomerated. (Note that when the polymerized slurry of this stage was subjected to azeotropic distillation of water and n-heptane using an oil bath at 120°C to distill off only water to the outside of the system and was subsequently dried through n-heptane evaporation, the resulting completely spherical primary particles had a median particle size of 60 μm, and an aspect ratio of 1.0.) The obtained water-absorbent resin has a median particle size of 355 μm and a water content of 6%. Table 1 shows the measurement results of each property.

Comparative Example 2

The same procedure as in Example 4 was repeated except that the aqueous monomer solution for the second stage was not added or the polymerization for the second stage was not carried out, the amount of azeotropic dehydration was changed to 141.8 g, and the amount of a 2% aqueous solution of ethylene glycol diglycidyl ether to be added after azeotropic dehydration was changed to 1.84 g, thereby obtaining 96.8 g of water-absorbent resin in which the comma-shaped bead-like primary particles are not agglomerated. The comma-shaped bead-like primary particles had a median particle size of 430 μm and a water content of 6%. Table 1 shows the measurement results of each property.

Comparative Example 3

160 g of acrylic acid was diluted with 10.3 g of water and neutralized by adding 266.6 g of a 25% by mass sodium hydroxide aqueous solution under cooling. 0.08 g of ethylene glycol diglycidyl ether, 0.016 g of sodium hypophosphite monohydrate, and 0.08 g of potassium persulfate were added to the solution and dissolved, thereby obtaining an aqueous monomer solution.

A portion (80.0 g) of the aqueous monomer solution prepared above was placed in a 200 mL beaker, and 0.60 g of polyoxyethylene octylphenyl ether phosphate (produced by Dai-ichi Kogyo Seiyaku Co., Ltd., Plysurf A210G, average degree of polymerization of oxyethylene group: about 7) as an dispersant, and 20.0 g of cyclohexane were added to the solution. The mixture was emulsified at 10,000 rpm using an emulsifying device (produced by PRIMIX Corporation, Mark II homogenizer) for 3 minutes, thereby obtaining an emulsified monomer solution.

624 g of cyclohexane was placed in a 4-necked round-bottomed flask (2 L capacity) equipped with a stirrer, a reflux condenser, a thermometer, and a nitrogen gas inlet tube; and 1.56 g of polyoxyethylene octylphenyl ether phosphate (produced by Dai-ichi Kogyo Seiyaku Co., Ltd., Plysurf A210G, average degree of polymerization of oxyethylene group: about 7) was added thereto as an dispersant, followed by stirring at 420 rpm for dispersion. After the inside of the flask was replaced with nitrogen, the temperature was raised to 80°C to flux cyclohexane. 50.3 g of the above-described emulsified monomer solution was added dropwise to the mixture at a rate of 6.6 g/min for 8 minutes. After the completion of dropwise addition, the mixture was left to stand at the same temperature for about 10 minutes. Subsequently, 357 g of the aqueous monomer solution prepared first was added dropwise to the mixture at a rate of 6.6 g/min for 54 minutes. After the completion of dropwise addition, the mixture was maintained at an internal temperature of 75°C for 30 minutes, followed by dehydration by azeotropy with cyclohexane until the water content in the produced resin particles was 7%.

After the completion of dehydration, the stirring was terminated, and the solid precipitated at the bottom of the flask was separated from the liquid by decantation. The obtained solid was dried under reduced pressure at 90°C, and cyclohexane and water were removed, thereby obtaining 189.5 g of water-absorbent resin in the form of the columnar primary particles having depressions and projections on the surface. The primary particles had a median particle size of 400 μm and a water content of 5%. Table 1 shows the measurement results of each property.

Comparative Example 4

In a reaction vessel formed by attaching a lid to a stainless-steel double arm kneader equipped with two sigma-shaped impellers and a jacket with a capacity of 5 L, 2.65 g of polyethylene glycol diacrylate (average added mole number of ethylene oxide: 38) was dissolved in 3,300 g of an aqueous solution of sodium acrylate having a neutralization ratio of 75% by mol (concentration of the unsaturated monomer: 38% by mass), thereby obtaining a reaction solution. Next, the reaction solution was deaerated under a nitrogen gas atmosphere for 30 minutes. Subsequently, 19.8 g of a 10% by mass sodium persulfate aqueous solution and 0.70 g of a 1% by mass L-ascorbic acid aqueous solution were added to the reaction solution. Thereby, the polymerization was started about 1 minute later. The polymerization was carried out at 20 to 95°C while crushing the produced gel, and a water-containing gel-like crosslinked polymer was taken out 50 minutes after the start of the polymerization. The obtained water-containing gel-like crosslinked polymer was broken into small pieces of a diameter of about 5 mm or less. The small pieces of the water-containing gel-like crosslinked polymer were spread on wire mesh (JIS-standard sieve having an opening of 300 $\mu$m), and dried by a hot-air dryer set at 180°C for 90 minutes. Next, the polymer was crushed using a roll crusher and further passed through a JIS-standard sieve having an opening of 850 $\mu$m, thereby obtaining a water-absorbent resin precursor.

100 g of the water-absorbent resin precursor was weighed out in a 1 L separable flask equipped with stirring impellers. While stirring the precursor, a cross-linking agent containing a mixture of ethylene glycol diglycidyl ether (0.03 g), propylene glycol (0.9 g), and water (3 g) was added by spraying. Subsequently, the flask was immersed in a hot-water bath at 190°C and heat-treated for 45 minutes. The sample after heating was sorted by a JIS-standard sieve having an opening of 850 $\mu$m, thereby obtaining 98.5 g of crushed water-absorbent resin. The obtained water-absorbent resin had a median particle size of 300 $\mu$m and a water content of 1%. Table 1 shows the measurement results of each property.

[Table 1]

| | | Examples | | | | | Comparative Examples | | | |
|---|---|---|---|---|---|---|---|---|---|---|
| | | 1 | 2 | 3 | 4 | 5 | 1 | 2 | 3 | 4 |
| Primary Particles | Median particle size | 190 | 280 | 110 | 430 | 80 | 60 | 430 | 400 | 300 |
| | Aspect ratio | 1.9 | 1.4 | 1.6 | 5.5 | 2.2 | 1.0 | 5.5 | 3.2 | 2.3 |
| Secondary Particles | Median particle size | 600 | 720 | 470 | 900 | 390 | 355 | - | - | - |
| | Aspect ratio | 1.4 | 1.2 | 1.3 | 1.8 | 1.7 | 1.4 | - | - | - |
| | Homogeneity of the particle size | 1.6 | 1.8 | 1.5 | 1.9 | 1.7 | 1.6 | 2.1 | 2.4 | 2.9 |
| Water absorption capacity (g/g) | | 60 | 46 | 56 | 65 | 52 | 59 | 60 | 58 | 57 |
| Water absorption capacity of saline solution under load | | 18 | 25 | 21 | 16 | 23 | 19 | 16 | 13 | 17 |
| Properties After Collision Test | (Median particle size) | (540) | (630) | (385) | (720) | (335) | (315) | (335) | (340) | (235) |
| | Particle strength | 90% | 88% | 82% | 80% | 86% | 89% | 78% | 85% | 78% |
| | (Water absorption capacity under load) | (16) | (21) | (18) | (13) | (20) | (15) | (11) | (10) | (13) |
| | Retention rate | 89% | 84% | 86% | 81% | 87% | 79% | 69% | 77% | 76% |
| Powder flow index | | 120 | 110 | 140 | 100 | 130 | 120 | 110 | 69 | 64 |
| Index of adhesion to fibers | | 80% | 83% | 71% | 83% | 76% | 47% | 32% | 64% | 68% |

Various properties, shown in Table 1, of the water-absorbent resins obtained in the Examples and Comparative Examples were measured by the following methods.

(Water Absorption Capacity)

500 g of a 0.9% by mass saline was placed in a 500 mL beaker; and 2.0 g of water-absorbent resin was added thereto, followed by stirring for 60 minutes. The contents in the beaker was filtered through a JIS-standard sieve having an opening of 75 $\mu$m whose mass Wa (g) has been measured in advance. The sieve was inclined at an angle of about 30 degrees relative to the horizon, and left to stand in that state for 30 minutes so as to filter out excess water. A mass Wb (g) of the sieve having water-absorbing gel therein was measured, and the water absorption capacity was determined by the following formula.

$$\text{Water absorption capacity (g/g)} = (Wb-Wa)/2.0$$

(Median Particle Size of the Primary Particles)

0.25 g of amorphous silica (Degussa Japan, product number: Sipernat 200) was mixed as a lubricant with 50 g of a water-absorbent resin.

JIS-standard sieves having openings of 500 $\mu$m, 355 $\mu$m, 250 $\mu$m, 180 $\mu$m, 106 $\mu$m, 75 $\mu$m, and 38 $\mu$m, and a receiving tray were combined in that order. The above-mentioned water-absorbent resin was placed on the top sieve, and shaken using a Ro-tap sieve shaker for 20 minutes.

Next, the mass of the water-absorbent resin remaining on each sieve was calculated as the mass percentage relative to the total mass, and the mass percentage was integrated in descending order of particle size. Thereby, the relationship between the sieve opening and the integrated value of the mass percentage of the water-absorbent resin remaining on the sieve was plotted on a logarithmic probability paper. By connecting the plots on the probability paper with a straight line, the particle size corresponding to the 50% percentile of the integrated mass percentage was defined as the median particle size of the primary particles.

(Median Particle Size of the Secondary Particle)

0.5 g of amorphous silica (Degussa Japan, product number: Sipernat 200) was mixed as a lubricant with 100 g of a water-absorbent resin.

In this measurement, 7 consecutive sieves were selected from 13 JIS-standard sieves (openings of 2.36 mm, 1.7 mm, 1.4 mm, 850 $\mu$m, 600 $\mu$m, 500 $\mu$m, 355 $\mu$m, 300 $\mu$m, 250 $\mu$m, 180 $\mu$m, 106 $\mu$m, 75 $\mu$m, and 45 pm) for use.

Sieves of 600 $\mu$m, 500 $\mu$m, 355 $\mu$m, 300 $\mu$m, 250 $\mu$m, 180 $\mu$m, and 106 $\mu$m, and a receiving tray were combined in that order, and the above-mentioned water-absorbent resin was placed on the top sieve and shaken using a Ro-tap sieve shaker for 20 minutes.

Subsequently, the mass of the water-absorbent resin remaining on each sieve was calculated as the mass percentage relative to the total mass, and the mass percentage was integrated in descending order of particle size. Thereby, the relationship between the sieve opening and the integrated value of the mass percentage of the water-absorbent resin remaining on the sieve was plotted on a logarithmic probability paper. By connecting the plots on the probability paper with a straight line, the particle size corresponding to the 50% percentile of the integrated mass percentage was defined as the median particle size.

When the mass percentage of the water-absorbent resin remaining on either the top sieve or the bottom receiving tray is over 15.9%, the homogeneity described below cannot be accurately determined. Therefore, in that case, 7 consecutive sieves were reselected from the above-mentioned sieves, and the size distribution was measured again until the mass percentage of the water-absorbent resin remaining on the top sieve and the bottom receiving tray was 15.9% or less.

(Uniformity of Particle Size Distribution)

In the measurement of the median particle size of the secondary particles, a particle size (X1) corresponding to 15.9% percentile of the integrated mass percentage and a particle size (X2) corresponding to 84.1% percentile of the integrated mass percentage were determined, and the uniformity was obtained by the following formula.

$$\text{Uniformity} = \text{X1/X2}$$

Specifically, when the particle size distribution is narrow, the uniformity is close to 1, whereas when the particle size distribution is broad, the uniformity is greater than 1.

(Particle Size Retention Rate after Particle Collision Test)

The particle size retention rate in a particle collision test of the water-absorbent resin was determined by measuring the particle size distribution when the water-absorbent resin was collided against a collision plate using a test device X schematically shown in Fig. 2.

The test device X shown in Fig. 2 comprises a hopper (with a lid) 1, a pressurized air introduction tube 2, an injection nozzle 3, a collision plate 4, and a flowmeter 5. The pressurized air introduction tube 2 is introduced into the interior of the hopper 1, and the injection nozzle 3 is connected to the hopper 1. The pressurized air introduction tube 2 has an external diameter of 3.7 mm, and an internal diameter of 2.5 mm. The injection nozzle 3 has an external diameter of 8 mm, an internal diameter of 6 mm, and a length of 300 mm. The material of the collision plate 4 is SUS304, and has a thickness of 4 mm. The distance between the end of the injection nozzle 3 and the collision plate 4 is fixed at 10 mm. The flowmeter 5 is adjusted such that the flow rate of pressurized air is 50 m/s at the end of the injection nozzle 3.

In the thus-configured test device X, first, 100 g of water-absorbent resin 6 whose median particle size before collision (A1) has been measured in advance was placed in the hopper 1, and the hopper 1 was closed. Subsequently, the pressurized air having an adjusted pressure was introduced from the pressurized air introduction tube 2, and the water-absorbent resin 6 was injected from the injection nozzle 3 to the collision plate 4. The water-absorbent resin after injecting and colliding the entire amount was collected; and the particle size distribution was measured, thereby determining a median particle size after collision (A2). Using the obtained value, the particle size retention rate after a particle collision test was determined by the following formula:

$$\texttt{Particle Size Retention Rate After Particle Collision Test}$$
$$\texttt{(\%) = [A2/A1] × 100}$$

(Water Absorption Capacity of Saline Solution under load)

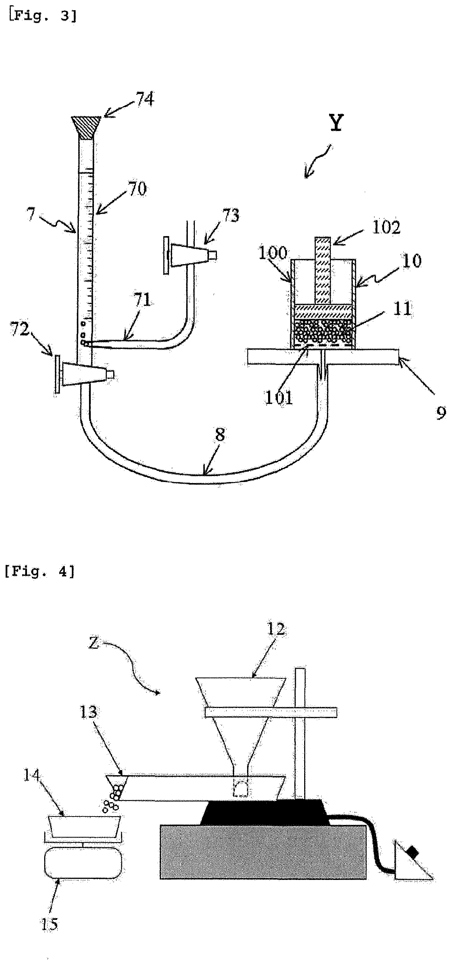

The water absorption capacity of saline solution under load of water-absorbent resin was measured using a measuring device Y schematically shown in Fig. 3. The measuring device Y shown in Fig. 2 comprises a burette section 7, a duct 8, a measuring board 9, and a measuring section 10 placed on the measuring board 9. The burette section 7 includes a rubber plug 74 connected to the top of a burette 70, and an air introduction tube 71 and a cock 72, which are connected to the lower portion of the burette 70. Further, the air introduction tube 71 has a cock 73 at the end thereof. The duct 8 is attached between the burette section 7 and the measuring board 9. The duct 8 has a diameter of 6 mm. A 2 mm-diameter hole is formed in the center of the measuring board 9, and the duct 8 is connected to the hole. The measuring section 10 has a cylinder 100 (made of Plexiglas), a nylon mesh 101 adhered to the bottom of the cylinder 100, and a weight 102. The cylinder 100 has an inner diameter of 20 mm. The nylon mesh 101 has an opening of 75 μm (200 mesh). Water-absorbent resin 11 is evenly spread over the nylon mesh 101 at the time of measurement. The weight 102 has a diameter of 19 mm and a mass of 119.6 g. The weight is placed on the water-absorbent resin 11 so that a load of 4.14 kPa can be applied to the water-absorbent resin 11.

Next, the measurement procedures are described. The measurement is carried out in a room at 25°C. First, the cock 72 and the cock 73 at the burette section 7 are closed, and 0.9% by mass saline adjusted to 25°C is poured from the top of the burette 70 and the top of the burette upper portion as plugged with the rubber plug 74. Thereafter, the cock 72 and the cock 73 at the burette section 7 are opened. Next, the height of the measuring board 9 was adjusted in such a manner that the level of surface of the 0.9% by mass saline flowing out from the duct opening in the center of the measuring board 9 and the upper surface of the measuring board 9 are at the same height.

Separately, 0.10 g of particles of the water-absorbent resin 11 is evenly dispersed over the nylon mesh 101 in the cylinder 100, and the weight 102 is placed on the water-absorbent resin 11, thereby preparing the measuring section 10. Subsequently, the measuring section 10 was placed in such a manner that its center is aligned with the duct opening in the center of the measuring board 9.

When the water-absorbent resin 11 started absorbing water, the amount of reduction in 0.9% by mass saline in the burette 100 (i.e., the volume of 0.9% by mass saline absorbed by the water-absorbent resin 11; indicated by Wc (ml)) was read off. The water absorption capacity of saline solution under load of the water-absorbent resin 11 at 60 minutes after the start of water absorption was determined by the following formula:

$$\texttt{Water absorption capacity of saline solution under load}$$
$$\texttt{(ml/g) = Wc/0.10}$$

(Retention Rate of Water Absorption Capacity under Load after Particle Collision Test)

100 g of water-absorbent resin whose water absorption capacity of saline solution under load before particle collision test (B1) has been measured in advance according to the method described in the above Water Absorption Capacity under load was subjected to a particle collision test according to the method described in the above Particle Size Retention Rate After Particle Collision Test. Using the collected water-absorbent resin, the water absorption capacity under load was again measured according to the method described in Water Absorption Capacity under load to determine a water absorption capacity under load after particle collision test (B2). Using the determined value, the retention rate of the water absorption capacity under load after particle collision test was determined by the following formula:

$$\text{Retention rate of water absorption capacity under load}$$
$$\text{after particle collision test (\%) = [B2/B1]} \times 100$$

(Aspect Ratio)

A scanning electron micrograph (SEM) of water-absorbent resin was taken. 50 particles were arbitrarily selected from the micrographs. The maximum length of each particle in a longitudinal direction was measured as the major axis, and the maximum length perpendicular to the major axis was measured as the minor axis. The average of the measured values of all particles was calculated, as well as the aspect ratio of the resin particles (major axis/minor axis ratio).

(Powder Flow Index)

The powder flow index was measured using a powder flowability measuring device Z schematically shown in Fig. 4. The powder flow index was calculated using a vibratory powder sample feeder (produced by Fritsch, product number: L-24). First, in a room at a temperature of 25°C and a relative humidity of 50 to 75%, the clearance between a hopper 12 and a V-shaped trough 13 of the feeder was fixed at 2 mm, and then the feeder was fixed in such a manner that the angle relative to the horizontal plane of the trough was $-0.5 \pm 0.5$ degrees. An electronic scale 15 (capable of measuring to 0.01 g) having a metal tray 14 thereon was placed under the end of the trough. Next, 200 g of salt (Wako Pure Chemical Industries, Ltd., special grade, median particle size: 550 $\mu$m) was fed into the hopper. Powder was allowed to flow by setting the feed rate to 5 (out of 10 levels) and the vibration frequency to high. The time from when the amount of powder accumulated on the tray was 50 g to when the amount of powder accumulated on the tray was 150 g was measured, and a transfer time T1 (seconds) taken by 100 g of salt from the hopper to the tray was measured. The time measured for the salt was about 150 seconds.

The same test was performed for the water-absorbent resin, and a transfer time T2 (seconds) taken by 100 g of water-absorbent resin was measured. The powder flow index of the water-absorbent resin was calculated by the following formula. Note that, in general, the speed of powder supply varies depending on the particle size, even if the production conditions are the same. Because the apparent supply time tends to be longer when powder has a smaller particle size, the flow index was corrected using a median particle size D1 of salt, and a median particle size D2 of water-absorbent resin.

$$\text{Powder flow index} = [(100/T2) \times (1/D2)]/[(100/T1) \times (1/D1)] \times 100$$

(Index of Adhesion to Fibers)

5.3 g of water-absorbent resin (Wd) and 2.2 g of crushed wood pulp were dry-blended. The resulting mixture was sprayed on tissue paper 15 cm $\times$ 12 cm in size, and pressed by applying a load of 196 kPa to the entire mixture for 30 seconds, thereby preparing an absorbent material. The absorbent material was carefully placed in the center of a JIS-standard sieve (internal diameter of 20 cm, opening of 1.18 mm) having a receiving tray, and the tissue paper was removed. An acrylic plate 16 cm $\times$ 12 cm in size was inserted into the mesh above the absorbent material. The acrylic plate was used to prevent twisting and bias of the absorbent material. Because the acrylic plate was held at 5 mm from the top of the mesh, no substantial load was applied to the absorbent material, and the acrylic plate did not move in the horizontal direction when the sieves were shaken. A lid was placed on top of the sieves, thereby forming a measuring unit.

This measuring unit was fixed to a shaker of a constant-temperature shaking water bath (professional thermo shaker produced by Tokyo Rikakikai Co., Ltd., product number: NTS2100), and shaken at 130 rpm in the horizontal direction for 15 minutes. Subsequently, the absorbent material was carefully turned over and shaken under the same conditions for 15 minutes.

From the water-absorbent resin and pulp dropped into the receiving tray, only water-absorbent resin was carefully collected, and the amount of dropped water-absorbent resin (We) was measured. The index of adhesion to fibers was determined by the following formula.

$$\text{Index of Adhesion to Fibers} = [Wd-We]/Wd \times 100$$

(Water Content)

2 g of the water-absorbent resin was precisely weighed out (Wf (g)) in a previously weighed aluminum foil case (No.8). The above sample was dried for 2 hours in a hot air oven (produced by ADVANTEC) set at an internal temperature of 105°C. Thereafter, the dried sample was allowed to cool in a desiccator, and the mass Wg (g) of the dried water-absorbent resin was determined. The water content of the water-absorbent resin was calculated by the following formula:

$$\texttt{Water content (\%) = [Wf-Wg]/Wf} \times \texttt{100}$$

Industrial Applicability

The production method of the present invention can produce a water-absorbent resin that is excellent in general absorption properties of the water-absorbent resin; that has an increased particle strength while having a moderate particle size; and that is excellent in powder flowability because it has a small aspect ratio while having excellent adhesion to fibers. The water-absorbent resin having such properties is suitably used for a thin absorbent material containing a high proportion of absorbent resin, and an absorbent article that uses the same.

Brief Description of Drawings

[Fig. 1] Fig. 1 is a schematic diagram showing the water-absorbent resin of the present invention.
[Fig. 2] Fig. 2 is a general schematic diagram of a device for performing a collision test.
[Fig. 3] Fig. 3 is a general schematic diagram of a device for measuring the water absorption capacity of saline solution under load.
[Fig. 4] Fig. 4 is a general schematic diagram of a device for measuring powder flowability.

Reference Symbol List

a Primary particles
b Water-absorbent resin
c Depressions
X Collision test device
1 Hopper
2 Pressurized air introduction tube
3 Injection nozzle
4 Collision plate
5 Flowmeter
6 Water-absorbent resin
Y Device for measuring the water absorption capacity of saline solution under load
7 Burette section
70 Burette
71 Air introduction tube
72 Cock
73 Cock
74 Rubber plug
8 Duct
9 Measuring board
10 Measuring section
100 Cylinder
101 Nylon mesh
102 Weight
11 Water-absorbent resin
Z Powder flowability measuring device
12 Hopper

13 V-shaped trough
14 Metal tray
15 Electronic scale

**Claims**

1.  A water-absorbent resin in the form of a secondary particle which is obtainable by agglomerating primary particles having an aspect ratio of 1.4 to 50 and a median particle size (d) of 50 to 600 $\mu$m,

    the secondary particle having an aspect ratio of 1.0 to 3.0 and a median particle size (D) of 100 to 2,000 $\mu$m, the particle aspect ratio being determined by measuring the maximum length of 50 particles arbitrarily selected from a scanning electron micrograph as the major axis and the maximum length perpendicular to the major axis as the minor axis, and calculating the average of the measured values and the aspect ratio as the ratio of the major axis to the minor axis.

2.  The water-absorbent resin according to claim 1, wherein the water-absorbent resin has a particle size uniformity of 1.0 to 2.2, obtained by the formula:

    $$Uniformity = X1/X2$$

    wherein X1 is a particle size corresponding to 15.9% percentile of the integrated mass percentage in the measurement of the median particle size of the secondary particles, a X2 is a particle size corresponding to 84.1% percentile of the integrated mass percentage.

3.  The water-absorbent resin according to claim 1 or 2, wherein the water-absorbent resin has a flow index of 70 to 200 and an index of adhesion to fibers of 50 to 100,

    wherein the flow index is determined as defined herein using a powder flowability measuring device, by measuring a transfer time T1 in seconds, taken by 100 g of a salt, and a transfer time T2 in seconds, taken by 100 g of the water-absorbent resin, and calculating the flow index according to the formula:

    $$Powder\ flow\ index = [(100/T2) \times (1/D2)]/[(100/T1) \times (1/D1)] \times 100$$

    wherein D1 represents the median particle size of the salt and D2 represents the median particle size of the water-absorbent resin,
    and wherein the index of adhesion to fibers is determined as defined herein by shaking a measuring unit comprising an absorbent material formed by a water-absorbent resin (Wd) and a crushed wood pulp in a shaker at 130 rpm for 15 minutes to measure the amount of dropped water-absorbent resin (We), and calculating the index of adhesion to fibers according to the formula:

    $$Index\ of\ Adhesion\ to\ Fibers = [Wd-We]/Wd \times 100.$$

4.  The water-absorbent resin according to any one of claims 1 to 3, wherein the primary particles have a form comprising a curved surface, selected from a comma-shaped bead-like shape, an oval-spherical shape, a sausage shape, and a rugby ball shape.

5.  An absorbent material comprising the water-absorbent resin according to any one of claims 1 to 4 and a hydrophilic fiber.

6.  An absorbent article including the absorbent material according to claim 5 between a liquid-permeable sheet and a liquid-impermeable sheet.

7.  A method for producing a water-absorbent resin comprising a secondary particle according to a reversed phase suspension polymerization method including steps 1 and 2 described below:

**EP 2 615 117 B2**

(1) step 1, in which a water-soluble ethylenically unsaturated monomer is subjected to a polymerization reaction in the presence of a thickener and a dispersion stabilizer to form a slurry in which primary particles having an aspect ratio of 1.1 to 200 are dispersed, and

(2) step 2, in which the slurry obtained in step 1 is cooled to precipitate the dispersion stabilizer, and then a water-soluble ethylenically unsaturated monomer is further added to perform a polymerization reaction, thereby agglomerating the primary particles dispersed in the slurry to form the water-absorbent resin in the form of the secondary particle,

the thickener being at least one member selected from the group consisting of hydroxyethyl cellulose, hydroxypropyl cellulose, methyl cellulose, carboxymethyl cellulose, polyacrylic acid, (partially) neutralized products of polyacrylic acid, polyethylene glycol, polyacrylamide, polyethylene imine, dextrin, sodium alginate, polyvinyl alcohol, polyvinyl pyrrolidone, and polyethylene oxide,

the dispersion stabilizer being a surfactant and a polymeric dispersion agent,

the surfactant being at least one member selected from the group consisting of sucrose fatty acid esters, polyglycerol fatty acid esters, sorbitan fatty acid esters, polyoxyethylene sorbitan fatty acid esters, polyoxyethylene glycerol fatty acid esters, sorbitol fatty acid esters, polyoxyethylene sorbitol fatty acid esters, polyoxyethylene alkyl ethers, polyoxyethylene alkylphenyl ethers, polyoxyethylene castor oil, polyoxyethylene cured castor oil, alkylallylformaldehyde condensed polyoxyethylene ethers, polyoxyethylene polyoxypropylene block copolymers, polyoxyethylene polyoxypropyl alkyl ethers, polyethylene glycol fatty acid esters, alkyl glucosides, N-alkyl glucone amides, polyoxyethylene fatty acid amides, polyoxyethylene alkylamines, phosphoric esters of polyoxyethylene alkyl ethers, and phosphoric esters of polyoxyethylene alkylallyl ethers, and

the polymeric dispersion agent being at least one member selected from the group consisting of maleic anhydride-modified polyethylene, maleic anhydride-modified polypropylene, maleic anhydride-modified ethylene-propylene copolymers, maleic anhydride-modified EPDM (ethylene-propylene-diene terpolymer), maleic anhydride-modified polybutadiene, ethylene-maleic anhydride copolymers, ethylene-propylene-maleic anhydride copolymers, butadiene-maleic anhydride copolymers, oxidized polyethylene, ethylene-acrylic acid copolymers, ethyl cellulose, and ethyl hydroxyethyl cellulose.

**Patentansprüche**

1.  Ein wasserabsorbierendes Harz in Form eines Sekundärteilchens, erhältlich durch Agglomerieren von Primärteilchen mit einem Seitenverhältnis von 1,4 bis 50 und einer mittleren Teilchengröße (d) von 50 bis 600 um,

    wobei das Sekundärteilchen ein Seitenverhältnis von 1,0 bis 3,0 und eine mittlere Teilchengröße (D) von 100 bis 2.000 um aufweist,
    wobei das Teilchen-Seitenverhältnis bestimmt wird durch Messen der maximalen Länge von 50 Teilchen, beliebig ausgewählt aus einer Rasterelektronenmikroskopaufnahme, als die Hauptachse und der maximalen Länge senkrecht zur Hauptachse als die Nebenachse und Berechnen des Durchschnitts der gemessenen Werte und des Seitenverhältnisses als das Verhältnis der Hauptachse zur Nebenachse.

2.  Das wasserabsorbierende Harz nach Anspruch 1, wobei das wasserabsorbierende Harz eine Einheitlichkeit der Teilchengröße von 1,0 bis 2,2 aufweist, erhalten durch die Formel:

$$\text{Einheitlichkeit} = X1/X2$$

    wobei X1 eine Teilchengröße ist, die dem 15,9%-Perzentil des integrierten Massenprozentanteils bei der Messung der mittleren Teilchengröße der Sekundärteilchen entspricht, ein X2 eine Teilchengröße ist, die dem 84,1%-Perzentil des integrierten Massenprozentanteils entspricht.

3.  Das wasserabsorbierende Harz nach Anspruch 1 oder 2, wobei das wasserabsorbierende Harz einen Fließindex von 70 bis 200 und einen Haftindex an Fasern von 50 bis 100 aufweist,

    wobei der Fließindex bestimmt wird wie hier definiert, unter Verwendung einer Messvorrichtung für die Pulverfließfähigkeit, indem eine Transferzeit T1 in Sekunden, bezogen auf 100 g eines Salzes, und eine Transferzeit T2 in Sekunden, bezogen auf 100 g des wasserabsorbierenden Harzes, gemessen und der Fließindex gemäß der Formel berechnet wird:

$$\text{Pulverfließindex} = [(100/T2) \times (1/D2)]/[(100/T1) \times (1/D1)] \times 100$$

wobei D1 die mittlere Teilchengröße des Salzes darstellt und D2 die mittlere Teilchengröße des wasserabsorbierenden Harzes darstellt,

und wobei der Haftindex an Fasern bestimmt wird wie hier definiert, durch Schütteln einer Messeinheit, die ein absorbierendes Material umfasst, das durch ein wasserabsorbierendes Harz (Wd) und eine zerkleinerte Holzpulpe gebildet wird, in einem Schüttler bei 130 U/min für 15 Minuten, um die Menge an abgetropftem wasserabsorbierendem Harz (We) zu messen, und Berechnen des Haftindex an Fasern gemäß der Formel:

$$\text{Haftindex an Fasern} = [Wd-We]/Wd \times 100.$$

4. Das wasserabsorbierende Harz nach einem der Ansprüche 1 bis 3, wobei die Primärteilchen eine Form aufweisen, die eine gekrümmte Oberfläche umfasst, ausgewählt aus einer kommaförmigen, perlenartigen Form, einer ovalsphärischen Form, einer Wurstform und einer Rugbyballform.

5. Ein absorbierendes Material, umfassend das wasserabsorbierende Harz nach einem der Ansprüche 1 bis 4 und eine hydrophile Faser.

6. Ein absorbierender Gegenstand, der das absorbierende Material gemäß Anspruch 5 zwischen einer flüssigkeitsdurchlässigen Lage und einer flüssigkeitsundurchlässigen Lage beinhaltet.

7. Ein Verfahren zur Herstellung eines wasserabsorbierenden Harzes, umfassend ein Sekundärteilchen, nach einem Umkehrphasen-Suspensionspolymerisationsverfahren, das die unten beschriebenen Schritte 1 und 2 einschließt:

(1) Schritt 1, in dem ein wasserlösliches ethylenisch ungesättigtes Monomer einer Polymerisationsreaktion in Gegenwart eines Verdickungsmittels und eines Dispersionsstabilisators unterzogen wird, um einen Slurry zu bilden, in dem Primärteilchen mit einem Seitenverhältnis von 1,1 bis 200 dispergiert sind, und

(2) Schritt 2, in dem der in Schritt 1 erhaltene Slurry gekühlt wird, um den Dispersionsstabilisator auszufällen, und dann ein wasserlösliches ethylenisch ungesättigtes Monomer weiter zugegeben wird, um eine Polymerisationsreaktion durchzuführen, wodurch die in dem Slurry dispergierten Primärteilchen agglomeriert werden, um das wasserabsorbierende Harz in Form des Sekundärteilchens zu bilden,

wobei das Verdickungsmittel mindestens ein Element ist, ausgewählt aus der Gruppe bestehend aus Hydroxyethylcellulose, Hydroxypropylcellulose, Methylcellulose, Carboxymethylcellulose, Polyacrylsäure, (teilweise) neutralisierten Produkten von Polyacrylsäure, Polyethylenglykol, Polyacrylamid, Polyethylenimin, Dextrin, Natriumalginat, Polyvinylalkohol, Polyvinylpyrrolidon und Polyethylenoxid,

wobei der Dispersionsstabilisator ein oberflächenaktives Mittel und ein polymeres Dispersionsmittel ist,

wobei das oberflächenaktive Mittel mindestens ein Element ist, ausgewählt aus der Gruppe bestehend aus Saccharosefettsäureestern, Polyglycerinfettsäureestern, Sorbitanfettsäureestern, Polyoxyethylensorbitanfettsäureestern, Polyoxyethylenglycerinfettsäureestern, Sorbitfettsäureestern, Polyoxyethylensorbitfettsäureestern, Polyoxyethylenalkylethern, Polyoxyethylenalkylphenylethern, Polyoxyethylenrizinusöl, Polyoxyethylen- gehärtetem Rizinusöl, Alkylallylformaldhydkondensierten Polyoxyethylenethern, Polyoxyethylen-Polyoxypropylen-Blockcopolymeren, Polyoxyethylen-Polyoxypropylalkylethern, Polyethylenglykolfettsäureestern, Alkylglucosiden, N-Alkylgluconamiden, Polyoxyethylen-Fettsäureamiden, Polyoxyethylen-Alkylaminen, Phosphorsäureestern von Polyoxyethylen-Alkylethern und Phosphorsäureestern von Polyoxyethylen-Alkylallylethern und

wobei das polymere Dispersionsmittel mindestens ein Element ist, ausgewählt aus der Gruppe bestehend aus Maleinsäureanhydrid-modifiziertem Polyethylen, Maleinsäureanhydrid-modifiziertem Polypropylen, Maleinsäureanhydrid-modifizierten Ethylen-Propylen-Copolymeren, Maleinsäureanhydrid-modifiziertem EPDM (Ethylen-Propylen-Dien-Terpolymer), Maleinsäureanhydrid-modifiziertem Polybutadien, Ethylen-Maleinsäureanhydrid-Copolymeren, Ethylen-Propylen-Maleinsäureanhydrid-Copolymeren, Butadien-Maleinsäureanhydrid-Copolymeren, oxidiertem Polyethylen, EthylenAcrylsäure-Copolymeren, Ethylcellulose und Ethylhydroxyethylcellulose.

**Revendications**

1. Résine absorbant l'eau sous la forme d'une particule secondaire qui susceptible d'être obtenue par agglomération de particules primaires ayant un rapport d'aspect de 1,4 à 50 et une granulométrie médiane (d) de 50 à 600 μm,

   la particule secondaire ayant un rapport d'aspect de 1,0 à 3,0 et une granulométrie médiane (D) de 100 à 2000 μm,
   le rapport d'aspect des particules étant déterminé par mesure de la longueur maximale de 50 particules choisies arbitrairement sur une micrographie électronique à balayage, considérée être l'axe majeur, et de la longueur maximale perpendiculaire à l'axe majeur, considérée être l'axe mineur, et calcul de la moyenne des valeurs mesurées et du rapport d'aspect sous la forme du rapport de l'axe majeur à l'axe mineur.

2. Résine absorbant l'eau selon la revendication 1, laquelle résine absorbant l'eau a une uniformité de granulométrie de 1,0 à 2,2, obtenue par la formule :

$$\text{uniformité} = X1/X2$$

   dans laquelle X1 est la granulométrie correspondant au 15,9ème percentile du pourcentage massique intégré dans la mesure de la granulométrie médiane des particules secondaires, et X2 est la granulométrie correspondant au 84,1ème percentile du pourcentage massique intégré.

3. Résine absorbant l'eau selon la revendication 1 ou 2, laquelle résine absorbant l'eau a un indice d'écoulement de 70 à 200 et un indice d'adhérence aux fibres de 50 à 100,

   dans laquelle l'indice d'écoulement est déterminé comme défini ici, par utilisation d'un dispositif de mesure de fluidité d'une poudre, par mesure du temps de transfert T1 en secondes, pris pour 100 g d'un sel, et du temps de transfert T2 en secondes, pris pour 100 g de la résine absorbant l'eau, et calcul de l'indice d'écoulement conformément à la formule :

$$\text{indice d'écoulement d'une poudre} = [(100/T2) \times (1/D2)] / [(100/T1) \times (1/D1)] \times 100$$

   dans laquelle D1 représente la granulométrie médiane du sel et D2 représente la granulométrie médiane de la résine absorbant l'eau,
   et dans laquelle l'indice d'adhérence aux fibres est déterminé comme défini ici par secouage d'une unité de mesure comprenant un matériau absorbant formé par une résine absorbant l'eau (Wd) et une pâte de bois broyée dans un secoueur à 130 t/min pendant 15 minutes pour que soit mesurée la quantité de résine absorbant l'eau ayant chuté (We), et calcul de l'indice d'adhérence aux fibres conformément à la formule :

$$\text{indice d'adhérence aux fibres} = [Wd-We]/Wd \times 100.$$

4. Résine absorbant l'eau selon l'une quelconque des revendications 1 à 3, dans laquelle les particules primaires ont une forme comprenant une surface incurvée, choisie parmi une forme de perle en forme de virgule, une forme ovale sphérique, une forme de saucisse, et une forme de ballon de rugby.

5. Matériau absorbant comprenant la résine absorbant l'eau de l'une quelconque des revendications 1 à 4 et une fibre hydrophile.

6. Article absorbant contenant le matériau absorbant de la revendication 5 entre une feuille perméable aux liquides et une feuille imperméable aux liquides.

7. Procédé pour produire une résine absorbant l'eau comprenant une particule secondaire selon un procédé de polymérisation en suspension à inversion de phases, comprenant les étapes 1 et 2 décrites ci-dessous :

   (1) étape 1, dans laquelle un monomère à insaturation éthylénique soluble dans l'eau est soumis à une réaction de polymérisation en présence d'un épaississant et d'un stabilisant de dispersion pour former une suspension dans laquelle des particules primaires ayant un rapport d'aspect de 1,1 à 200 sont dispersées, et

(2) étape 2, dans laquelle la suspension obtenue dans l'étape (1) est refroidie pour que le stabilisant de dispersion précipite, et ensuite un monomère à insaturation éthylénique soluble dans l'eau est en outre ajouté pour que s'effectue une réaction de polymérisation, avec pour résultat l'agglomération des particules primaires dispersées dans la suspension pour que se forme la résine absorbant l'eau sous la forme de la particule secondaire,

l'épaississant étant au moins un membre choisi dans le groupe constitué par l'hydroxyéthylcellulose, l'hydroxypropylcellulose, la méthylcellulose, la carboxyméthylcellulose, le poly(acide acrylique), les produits (partiellement) neutralisés de poly(acide acrylique), le polyéthylèneglycol, le polyacrylamide, la polyéthylène-imine, la dextrine, l'alginate de sodium, le poly(alcool vinylique), la polyvinylpyrrolidone, et le poly(oxyde d'éthylène),

le stabilisant de dispersion étant un tensioactif et un agent de dispersion polymère,

le tensioactif étant au moins un membre choisi dans le groupe constitué par les esters d'acide gras et de saccharose, les esters d'acide gras et de polyglycérol, les esters d'acide gras et de sorbitan, les esters d'acide gras et de sorbitan polyoxyéthylénés, les esters d'acide gras et de glycérol polyoxyéthylénés, les esters d'acide gras et de sorbitol, les esters d'acide gras et de sorbitol polyoxyéthylénés, les alkyléthers polyoxyéthylénés, les alkylphényléthers polyoxyéthylénés, l'huile de ricin polyoxyéthylénée, l'huile de ricin hydrogénée polyoxyéthylénée, les éthers polyoxyéthylénés condensés avec un alkylallylformaldéhyde, les copolymères séquencés de poly(oxyde d'éthylène) et de poly(oxyde de propylène), les polyoxypropylalkyléthers polyoxyéthylénés, les esters d'acide gras et de polyéthylèneglycol, les alkylglucosides, les N-alkylgluconamides, les amides d'acide gras polyoxyéthylénés, les alkylamines polyoxyéthylénées, les esters d'acide phosphorique et d'alkyléthers polyoxyéthylénés, et les esters d'acide phosphorique et d'alkylallyléthers polyoxyéthylénés, et

l'agent de dispersion polymère étant au moins un membre choisi dans le groupe constitué par le polyéthylène modifié par de l'anhydride maléique, le polypropylène modifié par de l'anhydride maléique, les copolymères d'éthylène-propylène modifiés par de l'anhydride maléique, l'EPDM (terpolymère d'éthylène-propylène-diène) modifié par de l'anhydride maléique, le polybutadiène modifié par de l'anhydride maléique, les copolymères d'éthylène-anhydride maléique, les copolymères d'éthylène-propylène-anhydride maléique, les copolymères de butadiène-anhydride maléique, le polyéthylène oxydé, les copolymères d'éthylène-acide acrylique, l'éthylcellulose, et l'éthylhydroxyéthylcellulose.

[Fig. 1]

[Fig. 2]

[Fig. 3]

[Fig. 4]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H9510889 B
- JP H857311 B
- JP H1294703 B
- JP H2153907 B
- JP H3227301 B
- JP H517509 B
- JP H543610 B
- JP HII140194 B
- JP 2008533213 A
- JP H2196802 B
- JP 2002284803 A
- JP 2004002891 A
- EP 2011803 A1
- EP 2184300 A1
- EP 2674441 A1
- WO 2006014031 A1